# FASCICULE DE BREVET EUROPEEN

(11) **EP 2 222 268 B1**
(45) Date de publication et mention de la délivrance du brevet: **02.05.2012**
(21) Numéro de dépôt: 08855872.1
(22) Date de dépôt: 24.11.2008
(51) Int. Cl.: C12M 1/00, C12M 1/36

(54) **RACCORDEMENT D'UN ACCESSOIRE A UN RECIPIENT**
VERBINDUNG EINES ZUBEHÖRS MIT EINEM GEFÄSS
CONNECTION OF AN ACCESSORY TO A VESSEL

(30) Priorité: 27.11.2007 FR 0708293
(43) Date de publication de la demande: 01.09.2010
(73) Titulaire: Sartorius Stedim Biotech S.A., 13400 Aubagne (FR)
(72) Inventeur: BERNARD, Frédéric, F-83740 La Cadiere D'azur (FR); CHEVALIER, Eric, F-75015 Paris (FR)
(74) Mandataire: Derambure, Christian
(86) Numéro de dépôt international: PCT/FR2008/052117
(87) Numéro de publication internationale: WO 2009/071829

(56) Documents cités:
- DE-A1-102004 015 703
- DE-A1-102006 005 533
- DE-C1- 4 207 845
- US-A1- 2005 239 198
- US-A1- 2005 239 199

## Description

L'invention concerne le raccordement d'un accessoire à un récipient.

Elle vise plus spécialement un dispositif de raccordement à un récipient d'un accessoire dont la partie proximale active est apte à être mis en communication avec l'intérieur du récipient par l'intermédiaire d'une ouverture du récipient ; un premier ensemble comprenant un dispositif de raccordement et un accessoire ; un second ensemble comprenant le premier ensemble et un récipient comportant une ouverture dans laquelle est monté le dispositif de raccordement ; un procédé de montage du second ensemble ; et un procédé de mise en oeuvre du second ensemble.

Le récipient en question est en l'espèce un récipient de stockage et/ou de traitement d'un contenu tel qu'un produit biopharmaceutique. Un tel récipient s'entend en l'espèce d'un récipient rigide réutilisable ou d'un récipient souple à usage unique tel qu'une poche.

Cette poche peut être une poche dite 2D, telle que celle commercialisée par Sartorius Stedim Biotech sous la marque Flexboy®, dont le volume, typiquement, peut être compris entre 50 millilitres et 50 litres.

Le document WO 00/04131 décrit un tel récipient souple dont le volume peut dépasser, et généralement dépasse, 50 litres. Un tel récipient est couramment appelé poche 3D. Il est alors connu qu'à cette poche sont associés des moyens rigides de maintien extérieur de la poche, au moins lorsqu'elle comporte son contenu.

Un produit biopharmaceutique s'entend en l'espèce d'un (ou plusieurs) produit issu de la biotechnologie - milieux de cultures, cultures cellulaires, solutions tampon, liquides de nutrition artificielle, produits sanguins et dérivés de produits sanguins - ou un produit pharmaceutique ou plus généralement un produit destiné à être utilisé dans le domaine médical. Un tel produit est sous forme liquide, pâteuse, poudreuse, en une ou plusieurs phases, homogènes ou non. L'invention s'applique également à des produits autres que biopharmaceutiques, selon la définition qui vient d'en être donnée, mais qui soumis à des exigences analogues en ce qui concerne leur stockage ou leur traitement.

L'accessoire s'entend en l'espèce d'une sonde pour mesurer des paramètres relatifs au contenu du récipient, comme la pression, le pH, la température, la colorimétrie, la conductimétrie, etc., ou encore un tube de remplissage ou un tube de vidange. L'invention s'applique également à des accessoires autres, mais qui ont vocation à être raccordés à un récipient en étant mis en communication avec l'intérieur de celui-ci.

Dans le domaine de la biopharmacie, il est usuel d'utiliser les récipients comme lieu pour effectuer des réactions chimiques ou biologiques, et le cas échéant les suivre et/ou les contrôler, ou encore comme moyen de stockage. Afin d'éviter la pénétration de germes à l'intérieur du récipient, il importe que l'environnement soit clos, stérile et aseptique, sans contact avec le milieu extérieur.

Les réactions doivent, en général, se dérouler dans des conditions déterminées et contrôlées (pression, pH, température, colorimétrie, conductimétrie, etc.) ou le stockage réalisé dans des conditions maîtrisées. Il est donc nécessaire d'effectuer plus ou moins fréquemment des mesures ou des contrôles de paramètres caractérisant le produit contenu dans le récipient. Ces mesures doivent être effectuées dans des conditions aseptiques, pour les raisons indiquées précédemment.

Le document DE 10 2004 015703 décrit un dispositif de raccordement d'un accessoire à un récipient comprenant deux éléments pourvus de moyens de guidage et de crochets. Ce dispositif ne comprend ni troisième élément auquel est destiné à être fixé l'accessoire et qui comporte une tête de protection et une base, ni chambre.

Le document DE 42 07 845 décrit un dispositif de support d'électrode d'analyse, comportant un tube extérieur fixe, un tube intérieur mobile à coulissement et un ensemble monté transversalement incluant un piston monté dans un carter, cet ensemble pouvant former une entrée étanche pour le tube intérieur, de manière à pouvoir retirer l'électrode du récipient dans lequel elle est destinée à être placée, sans perdre le contenu de celui-ci. Ce dispositif ne vise pas la constitution d'une chambre fonctionnelle.

Le document WO 86/07 151 décrit un dispositif de maintenance pour le calibrage et le nettoyage automatique d'une sonde faisant partie d'un système de mesure en continu de processus chimique ou biologique, consistant à analyser des paramètres du fluide à investiguer.

Le document DE 25 57 542 décrit un dispositif de mesure pour des valeurs électriques de milieux s'écoulant, avec des détecteurs de mesure disposés dans des enceintes de passage, dans lequel le passage d'un corps de soupape forme l'enceinte de passage et le détecteur est monté dans ce dernier.

Le document US2005/0239199 décrit une sonde selon un agencement particulier.

Le document US2005/0239198 décrit un raccordement stérile et aseptique au moyen de deux éléments, un premier élément fixé sur le récipient et un deuxième élément apte à être raccordé sur le premier élément. Le premier élément comporte, à son extrémité libre, un moyen de connexion pourvu d'un couvercle. Le second élément comprend une sonde disposée à l'intérieur d'un manchon flexible pourvu d'un moyen de connexion pourvu d'un couvercle, le moyen de connexion du deuxième élément étant destiné à être connecté au moyen de connexion du premier élément après retrait de leur couvercle respectif. La sonde est fixée au récipient en glissant le moyen de connexion du deuxième élément dans le moyen de connexion du premier élément. L'extrémité de la sonde est ensuite insérée à l'intérieur du récipient par compression du manchon, la sonde glissant à l'intérieur du premier élément jusqu'à atteindre l'intérieur du récipient.

Il existe donc un besoin pour assurer un raccordement étanche d'un accessoire à un récipient, une insertion aisée et rapide de l'accessoire dans le récipient et le cas échéant une calibration de l'accessoire lorsque celui-ci est une sonde, et une protection permanente de l'accessoire. Ce besoin doit être satisfait avec un dispositif simple et moyennant un procédé de mise en oeuvre facile et sans risque.

A cet effet, selon un premier aspect, l'invention vise un dispositif de raccordement à un récipient d'un accessoire dont la partie proximale active est apte à être mis en communication avec l'intérieur du récipient par l'intermédiaire d'une ouverture du récipient, le dispositif comprenant un premier élément, un deuxième élément, un troisième élément et une chambre, dans lequel :
■ le premier élément a pour fonctions :
   ○ d'assurer la fixation rigide du dispositif de raccordement au récipient,
   ○ de définir une voie de passage, entre l'intérieur et l'extérieur du récipient,
   ○ de servir de support pour le deuxième élément et de guide en translation axiale sur une course axiale C dont les deux fins de course correspondent à un premier état distal et à un second état proximal,
   ○ de contribuer à servir de guide pour le troisième élément,
   ○ de contribuer à délimiter la chambre,
■ le deuxième élément a pour fonctions :
   ○ de servir de support au troisième élément, lequel est fixé rigidement sur lui,
   ○ de former un élément mobile à coulissement axial sur la course C dont les deux fins de course correspondent au premier état distal et au second état proximal,
■ le troisième élément, auquel appartient une tête a pour fonctions :
   ○ de servir de support à l'accessoire, lequel est fixé rigidement sur lui,
   ○ de former un élément mobile à coulissement axial sur la course C dont les deux fins de course, correspondent au premier état distal et au second état proximal,
   ○ dans le premier état distal, d'assurer, grâce à la tête, la fermeture étanche de l'ouverture,
   ○ dans le second état proximal, d'assurer, grâce à la tête, une protection de la partie proximale active,
   ○ de contribuer à délimiter la chambre,
■ la chambre est dans le premier état distal fermée de façon étanche et, dans le second état proximal, en communication avec l'intérieur et ayant pour fonction d'y loger à l'intérieur ou d'y placer en périphérie la partie proximale active, et, le cas échéant,
   ○ d'y mettre un liquide de stockage,
   ○ d'y mettre un liquide de réglage, contrôle ou calibration de l'accessoire ou de constituer une chambre fermée de façon étanche dans lequel est placé l'accessoire en attente d'utilisation.

Selon une réalisation, dans le premier état distal, le deuxième élément est latéralement en regard du premier élément, sans être en regard d'une partie du premier élément attenante à son extrémité proximale une partie du deuxième élément saillant de l'extrémité distale du premier élément, et, dans le second état proximal, le deuxième élément est latéralement tout entier en regard du premier élément vers son extrémité distale, sans être en regard d'une partie plus courte axialement du premier élément attenante à son extrémité proximale.

Selon une réalisation, le troisième élément comporte outre la tête, une base et un corps,
■ la tête ayant pour fonctions :
   ○ dans le premier état distal, de fermer de façon étanche le premier élément,
   ○ dans le second état proximal, de contribuer à la protection de la partie proximale active,
■ la base ayant pour fonctions :
   ○ de servir de support à l'accessoire, lequel est fixé rigidement sur elle,
   ○ d'assurer le guidage de coulissement, avec étanchéité, du troisième élément sur et par rapport au premier élément,
   ○ de contribuer à délimiter la chambre,
■ le corps comportant une partie de liaison pourvue d'un alésage dans lequel l'accessoire est inséré et au moins un organe porteur, reliant rigidement la tête et la base.

Selon une réalisation, la base comporte :
■ dans sa partie extrême proximale, au moins une première bague, apte à pouvoir coulisser axialement, avec étanchéité, par sa face extérieure, sur et le long de la face intérieure de la paroi tubulaire du premier élément,
■ à sa partie extrême distale, une seconde bague, apte à être fixée rigidement par sa face extérieure, sur et contre la face intérieure de la paroi tubulaire du deuxième élément,
■ des faces intérieures des bagues étant aptes à permettre la fixation rigide, avec étanchéité, de l'accessoire.

Selon un deuxième aspect, l'invention vise un premier ensemble comprenant un dispositif de raccordement et un accessoire à usage unique, comportant une partie proximale active, fixé rigidement au troisième élément, la partie proximale active étant logée à l'intérieur ou placée en périphérie de la chambre.

Selon les réalisations, la partie distale de l'accessoire saille de façon distale de l'extrémité distale du deuxième élément ; et/ou à l'accessoire est associée fonctionnellement au moins une autre partie fonctionnelle, placée à côté ou à proximité de l'accessoire : et/ou la partie fonctionnelle est au moins un tube et plus spécialement au moins deux tubes, l'un d'amenée, l'autre de vidange.

Dans une réalisation telle que celle correspondant au cas où l'accessoire est une sonde, la partie fonctionnelle comporte préférentiellement au moins deux tubes, l'un d'amenée, et de calibration, l'autre de vidange et de rinçage.

Selon les réalisations, l'accessoire est au moins une sonde ou au moins un un tube.

Selon un troisième aspect, l'invention vise un second ensemble comprenant le premier ensemble et un récipient comportant une ouverture dans laquelle est monté le dispositif de raccordement.

Selon les réalisations, le récipient est rigide et réutilisable ou souple et à usage unique tel qu'une poche dite 3D.

Selon un quatrième aspect, l'invention vise un procédé de montage du second ensemble, dans lequel :
■ on dispose d'un récipient vide de contenu, de l'accessoire, et des éléments constitutifs du dispositif de raccordement,
■ dans une étape préliminaire, on fixe rigidement le premier élément sur le récipient à l'endroit de l'ouverture,
■ dans une autre étape préliminaire, on insère l'accessoire dans le troisième élément et on le fixe rigidement à celui-ci,
■ dans une deuxième étape ultérieure, on monte l'ensemble troisième élément + accessoire sur et dans le deuxième élément,
■ dans une troisième étape, on monte l'ensemble deuxième élément + troisième élément + accessoire sur et dans le premier élément, jusqu'à coopération des encoche(s) et ergot(s) correspondant au premier état distal.

Selon les réalisations, on prévoit une étape successive supplémentaire dans laquelle on aspire l'air (ou le gaz) se trouvant dans la chambre et on la vide de celui-ci ; une étape successive supplémentaire dans laquelle on injecte dans la chambre un liquide de stockage ; une étape successive supplémentaire dans laquelle on procède à une stérilisation de l'ensemble récipient + dispositif de raccordement + accessoire ; une étape successive supplémentaire dans laquelle on procède au conditionnement de l'ensemble récipient + dispositif de raccordement + accessoire.

Selon un cinquième aspect, dans un procédé de mise en oeuvre du second ensemble, on réalise des étapes opératoires liées spécifiquement au dispositif de raccordement et à l'accessoire comprenant les étapes opératoires suivantes :
- La chambre étant vide, faire passer l'ensemble dispositif de raccordement + accessoire du premier état distal au second état proximal.
- Puis, mettre en oeuvre l'accessoire.

Selon une réalisation, le procédé comporte une opération supplémentaire de réglage, de contrôle ou de calibration de l'accessoire.

Selon une réalisation, en ce qui concerne l'étape l'opération supplémentaire, il s'agit d'une calibration de l'accessoire, celui-ci étant alors une sonde auquel est associé un (ou comme exposé plus haut deux) tube(s), cette calibration étant réalisée au moyen de la chambre.

Selon une réalisation, on réalise une calibration unique ou plusieurs calibrations, telles qu'une calibration initiale, une calibration finale et, le cas échéant, une calibration intermédiaire.

On réalise une calibration alors que l'ensemble dispositif de raccordement + sonde + tube est dans le premier état distal et que la chambre est fermée de façon étanche.

Selon une réalisation, le procédé de mise en oeuvre comporte une étape opératoire préliminaire consistant à vider la chambre du liquide de stockage qui y a été mis lors de la fabrication, puis, le cas échéant la rincer.

On réalise une calibration par les étapes suivantes :
■ on injecte d'abord une solution de calibration dans la chambre fermée de façon étanche,
■ puis, la solution de calibration remplissant la chambre toujours fermée de façon étanche, et la partie proximale active étant en contact avec la solution de calibration, on calibre la sonde si cela est nécessaire et comme cela est requis,
■ puis, la chambre toujours fermée de façon étanche, on vidange de la solution de calibration,
■ puis on rince la chambre et on la vidange.

L'invention permet en particulier de pouvoir stocker, calibrer et utiliser une sonde à usage unique, dans des conditions stériles ou non.

L'invention est tout particulièrement applicable dans le domaine biopharmaceutique, par exemple pour des applications liées au mélange, pour les bioréacteurs à usage unique, pour la thermorégulation.

On décrit maintenant plusieurs modes de réalisation de l'invention à l'aide des dessins, dans lesquels :
- la figure 1 est une vue en coupe axiale d'un dispositif de raccordement, l'accessoire représenté schématiquement étant ici une sonde, le dispositif de raccordement et la sonde étant dans un premier état distal correspondant à une situation de stockage et/ou de calibration, une partie fonctionnelle qui ici est un tube de vidange et de calibration étant associée à l'accessoire, le dispositif de raccordement étant monté sur récipient dont la paroi périphérique n'est représentée que partiellement ;
- la figure 2 est une vue du dispositif de raccordement de la figure 1 lorsque le dispositif de raccordement, la sonde et le tube de vidange et de calibration sont dans un second état proximal correspondant à une situation d'utilisation de la sonde;
- la figure 3 est une vue en coupe axiale d'un premier élément du dispositif de raccordement de la figure 1 ;
- la figure 4 est une vue d'extrémité du premier élément de la figure 3, depuis son extrémité distale ;
- la figure 5 est une vue en coupe axiale d'un deuxième élément du dispositif de raccordement de la figure 1 montrant les ergots faisant partie des moyens de blocage de fin de course;
- la figure 6 est une vue en élévation du deuxième élément de la figure 5 montrant une patte élastique;
- la figure 7 est une vue d'extrémité du deuxième élément de la figure 5, depuis son extrémité distale ;
- la figure 8 est une vue en coupe axiale d'un troisième élément du dispositif de raccordement de la figure 1, montrant également l'accessoire à savoir la sonde et la partie fonctionnelle associée, à savoir le tube de vidange et de calibration;
- les figures 9 et 10 sont deux vues analogues respectivement aux figures 1 et 2, l'accessoire étant ici un tube de remplissage et/ou de vidange.

Un dispositif 1 est destiné au raccordement à un récipient 2 d'un accessoire 3 apte à être mis en communication avec l'intérieur 4 du récipient 2. On entend par « raccorder », le fait qu'originellement, le récipient 2 et l'accessoire 3 sont totalement distincts et séparés et que, grâce au dispositif 1, l'accessoire 3 est joint et relié au récipient et qu'un lien fonctionnel peut être établi entre le récipient 2, et son contenu, et l'accessoire 3. On dénomme « premier ensemble », l'ensemble comprenant le dispositif 1 de raccordement et l'accessoire 3 et « second ensemble », l'ensemble comprenant le premier ensemble 1, 3 et le récipient 2.

Conventionnellement, on désigne l'ensemble comprenant le récipient 2, le dispositif 1 de raccordement et l'accessoire 3 sous le nom de « conteneur fonctionnel » 5. Ce conteneur fonctionnel 5 est destiné au stockage et/ou au traitement d'un produit biopharmaceutique, désigné par la suite le contenu.

Selon les réalisations envisagées, le récipient 2 est rigide et réutilisable ou il est souple et à usage unique tel qu'une poche telle qu'une poche dite 2D ou une poche dite 3D, comme il a été défini plus haut.

Dans une réalisation, une telle poche 3D a une forme générale parallélépipédique, notamment cubique ou sensiblement cubique, ayant une paroi périphérique 6, formant au moins le fond et la partie latérale, et le plus souvent la partie supérieure, de manière que le récipient 2 soit de type fermé, et un ou plusieurs ports appropriés à la destination, ménagés sur la paroi 6. Dans une réalisation correspondant à un grand volume qui dépasse 50 l est peut atteindre 3.000 l, le récipient 2 est pliable.

Avec un tel récipient 2 souple, il est prévu que le conteneur 5 inclut en outre des moyens rigides de maintien extérieur. Ces moyens, dont l'existence est connue en soi, ne sont pas représentés. Il peut s'agir notamment d'un bac métallique ayant des parois pleines ou non.

La paroi 6 du récipient 2 est pourvue d'une l'ouverture 7 destinée au montage, transversalement par rapport à la paroi 6, du dispositif 1 de raccordement et de l'accessoire 3, de manière que ce dernier puisse être mis en communication avec l'intérieur 4 du récipient 2 et notamment à être mis en contact avec le contenu du récipient 2, alors même que l'accessoire 3 est situé au moins substantiellement dans l'extérieur 8 du récipient 2.

Ce qui vient d'être décrit dans le cas d'une poche 3D peut être transposé au cas d'une poche 2D et au cas d'un récipient 2 rigide.

Le dispositif 1, comme le plus souvent l'accessoire 3, présente un axe longitudinal 1a disposé au moins sensiblement perpendiculairement à la paroi 6.

L'intérieur 4 du récipient 2 est défini conventionnellement comme la zone interne au récipient 2, délimitée par l'enveloppe de la paroi 6, située du côté où se trouve la plus grande partie du contenu. L'extérieur 8 du récipient 2 est défini comme la zone se trouvant de l'autre côté de l'enveloppe de la paroi 6.

Les moyens rigides de maintien extérieur du récipient 2, lorsqu'ils sont prévus, comportent un passage pour le dispositif 1 de raccordement, situé en regard ou sensiblement en regard de l'ouverture 7.

Le terme « accessoire » signifie non pas que l'objet 3 qu'il désigne serait secondaire, ou simplement optionnel, mais que cet objet 3 est complémentaire et dans la dépendance étroite du récipient 2 et du contenu de celui-ci, en ayant une fonction accompagnant celle du récipient 2.

Dans le cadre de l'invention, l'accessoire 3 est préférentiellement à usage unique, tout comme le dispositif 1 de raccordement.

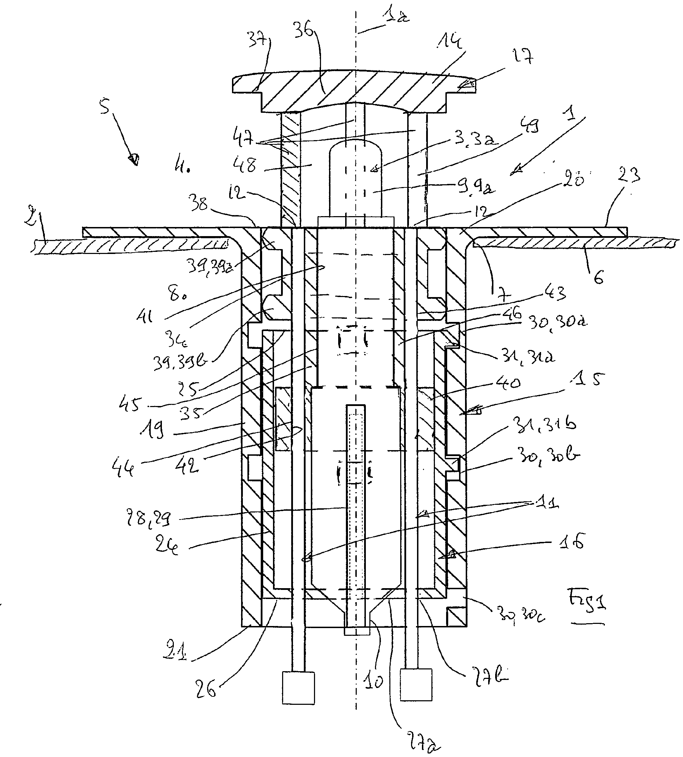

Selon une première réalisation (figures 1, 2 et 8), l'accessoire 3 est une sonde 3a comportant une partie proximale active 9a apte à mesurer, détecter, contrôler, enregistrer (par la suite mesurer) un ou plusieurs paramètres utiles se rapportant au contenu, comme notamment la pression, le pH, la température, la colorimétrie, la conductimétrie.

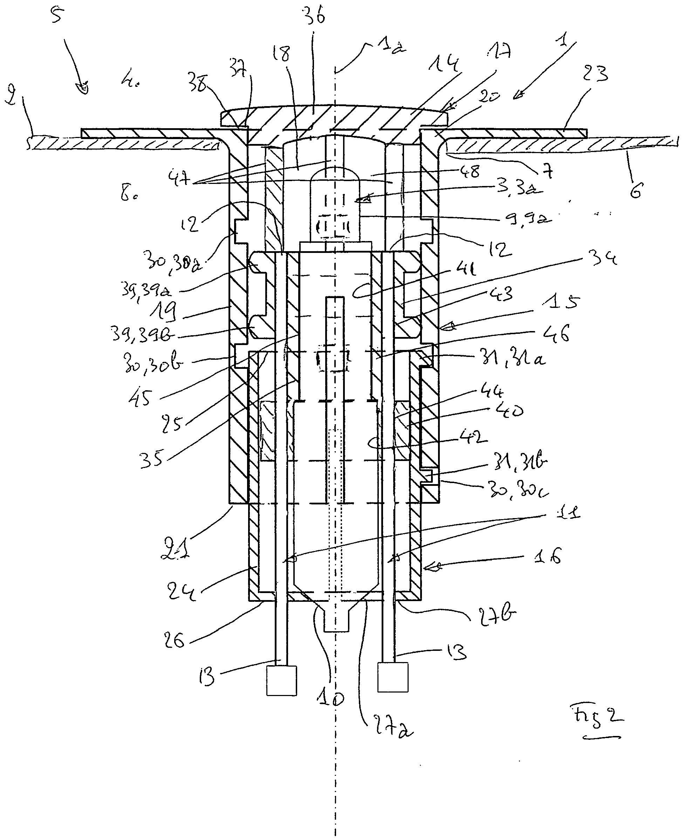

La partie active 9a de la sonde 3a est apte, lorsque nécessaire, à être mise en communication avec l'intérieur 4 du récipient 2, et plus spécialement à être mise en contact avec le contenu du récipient 2, afin de pouvoir jouer sa fonction de mesure. Dans ce cas, il est prévu, dans une réalisation représentée sur la figure 2, que la partie proximale active 9a est apte à être introduite dans l'intérieur 4 du récipient 2, en franchissant l'ouverture 7 de la paroi 6, pour aller au-delà de cette dernière, de manière à être en contact intime avec le contenu, dans une zone où celui-ci est homogène ou du moins suffisamment homogène, en ce qui concerne le paramètre en cause, avec le reste du contenu du récipient 2. L'ouverture 7 est alors préférentiellement située sur ou vers le fond du récipient 2, c'est-à-dire de manière à se trouver à un niveau plus bas que le niveau du contenu du récipient 2, même lorsque la quantité de contenu est faible et le niveau proche du fond du récipient 2.

Dans une autre réalisation, non représentée, la partie proximale active 9a de la sonde 3a est apte non pas, à proprement parler, à être introduite dans l'intérieur 4 du récipient 2, mais à être amenée en-deçà de la paroi 6 et à proximité de l'ouverture 7 sans toutefois la franchir, de manière à être en communication avec l'intérieur 4 du récipient 2.

Dans une autre réalisation, non représentée, la partie proximale active 9a de la sonde 3a est apte à être amenée dans l'ouverture 7, dans le plan de la paroi 6, de manière à être en communication avec l'intérieur 4 du récipient 2.

Dans ces différentes réalisations, situations et positions, la partie proximale active 9a de la sonde 3a (et la sonde 3a elle-même) est qualifiée d'insérée dans le récipient 2 ou dans l'intérieur 4 du récipient 2.

Dans d'autres réalisations, pouvant être combinées avec les précédentes, il est prévu non pas une mais plusieurs sondes 3a, soit pour plusieurs paramètres soit à différents endroits, notamment hauteur, du récipient 2.

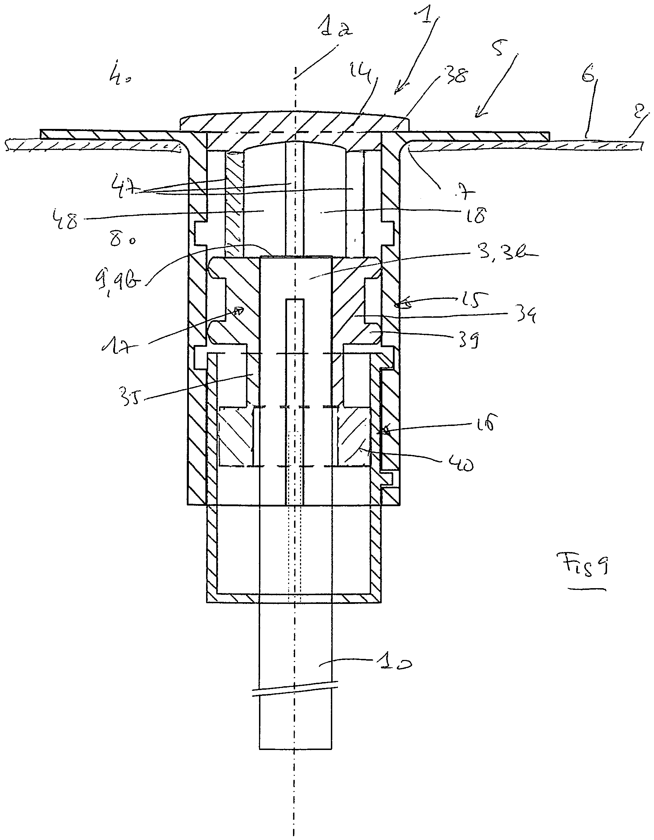

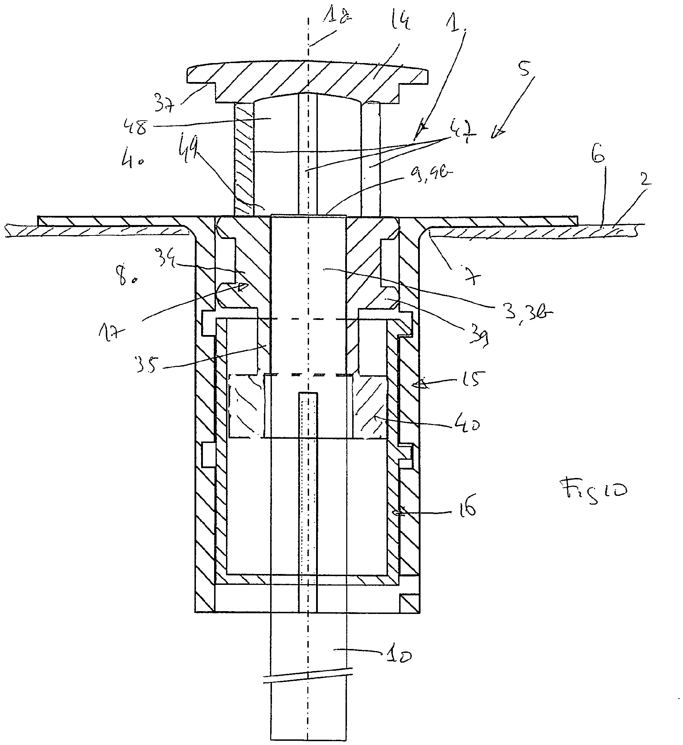

Selon une seconde réalisation (figures 9 et 10), l'accessoire 3 est un tube 3b d'amenée et/ou un tube 3b de vidange, du contenu du récipient 2 ou d'un ou plusieurs composants de celui-ci. Ce tube 3b comporte une partie proximale active 9b vers le récipient 2 consistant en une ouverture d'amenée si le tube est un, ou fait fonction de, tube d'amenée, en une ouverture de vidange si le tube est un, ou fait fonction de, tube de vidange, cette ouverture étant classiquement située à l'extrémité libre du tube 3b.

La partie proximale active 9b du tube 3b est apte, lorsque nécessaire, à être mise en communication avec l'intérieur 4 du récipient 2, afin de pouvoir jouer sa fonction d'amenée et/ou de vidange. A l'instar de la partie proximale active 9a de la sonde 3a, la partie proximale active 9b du tube 3b est alors apte soit à être amenée soit dans l'ouverture 7, dans le plan de la paroi 6 (figure 10) soit à être introduite dans l'intérieur 4 du récipient 2, en franchissant l'ouverture 7 de la paroi 6 (non représenté) soit à être amenée en-deçà de la paroi 6 et à proximité de l'ouverture 7 sans toutefois la franchir (non représenté). Ces différentes réalisations, situations et positions, correspondent à ce qui a été défini comme l'insertion de la partie proximale active 9b dans le récipient 2 ou dans l'intérieur 4 du récipient 2.

Lorsque l'accessoire 3 est un tube 3b d'amenée et/ou un tube 3b de vidange, l'ouverture 7 est située par rapport au fond du récipient 2 à la hauteur appropriée à la destination du tube 3b, soit à un niveau proche du fond du récipient 2 soit au contraire à un niveau plus ou moins éloigné du fond.

A l'instar de la sonde 3a, il peut être prévu plusieurs tubes 3b soit pour des composants différents soit à différents endroits, notamment hauteur, du récipient 2. D'autre part, un même tube peut avoir plusieurs fonctions : amenée, vidange et/ou plusieurs composants différents.

Selon d'autres réalisations, l'accessoire 3 est destiné à une autre fonction que la mesure, l'amenée ou la vidange. Au lieu d'être rigide, cet accessoire 3 peut être plus ou moins déformable. Il peut s'agir éventuellement d'un accessoire fonctionnel utile au contenu du récipient 2, par exemple un moyen de mélange.

De façon générale, l'accessoire 3 comporte une enveloppe extérieure de forme plus ou moins complexe, allongée, notamment de révolution à base circulaire. Sa partie active extrême proximale est référencée 9, et sa partie extrême distale est référencée 10. A cette partie extrême distale 10 peuvent être associés des moyens tels que conducteur électrique, tube...

Dans la réalisation représentée, l'accessoire 3 est disposé centralement par rapport à l'axe 1 a.

Selon des réalisations, à l'accessoire 3 proprement dit, est associé fonctionnellement au moins une autre partie fonctionnelle 11, placée à côté ou à proximité de l'accessoire 3 et s'étendant le long de l'axe 1a, en étant excentré par rapport à lui. Une telle partie fonctionnelle 11 comporte également une enveloppe extérieure, allongée, notamment de révolution à base circulaire. Dans la réalisation représentée, la partie fonctionnelle 11 est d'encombrement transversal nettement plus petit que celui de l'accessoire 3.

Une telle partie fonctionnelle 11 est, dans une réalisation, un ou plusieurs tube d'amenée et/ou de vidange associé à une sonde 3a afin d'assurer notamment sa calibration, comme il sera décrit par la suite. Dans un tel cas, le tube 11 comporte une partie extrême proximale 12 ouverte et une partie extrême distale 13 ouverte, pouvant se présenter, dans une réalisation, sous la forme d'un embout de connexion de type Luer ou filtre ou analogue.

Dans une réalisation spécifique, telle que celle correspondant au cas où l'accessoire 3 est une sonde pH 3a, la partie fonctionnelle 11 comporte préférentiellement au moins deux tubes, l'un d'amenée et le cas échéant de calibration, l'autre de vidange et le cas échéant de rinçage.

Le qualificatif « proximal » se réfère conventionnellement à ce qui, de façon relative, est proche, ou plus proche, du récipient 2, notamment de son intérieur 4. Le qualificatif « distal » se réfère inversement à ce qui, de façon relative, est éloigné, ou plus éloigné, du récipient 2, notamment de son intérieur 4. Ces qualificatifs sont utilisés pour repérer les parties constitutives du dispositif 1 de raccordement, de l'accessoire 3 et de la partie fonctionnelle 11. Il est entendu toutefois que le dispositif 1 de raccordement ou l'accessoire 3 ou la partie fonctionnelle 11 peut être considéré en soi, indépendamment du récipient 2, avant montage sur celui-ci. Ces qualificatifs sont utilisés également pour repérer la localisation des parties constitutives du dispositif 1 de l'accessoire 3 et de la partie fonctionnelle 11 par rapport au récipient 2 ou pour repérer leur position par rapport à celui-ci, si cette position n'est pas fixe. Ces qualificatifs sont utilisés enfin, conventionnellement, pour distinguer les deux états dans lesquels peuvent se trouver le dispositif 1 de raccordement, l'accessoire 3 et la partie fonctionnelle 11.

Le dispositif 1 de raccordement, l'accessoire 3 et notamment sa partie proximale active généralement référencée 9, la partie fonctionnelle 11 et notamment sa partie extrême distale 13, peuvent se trouver dans un de deux états possibles décrits par la suite, correspondant à deux positions relatives possibles par rapport au récipient 2, à deux manières d'être, fonctions, ou possibilité d'action. Ces états se comprennent lorsque le dispositif 1 de raccordement, l'accessoire 3 et la partie fonctionnelle 11 sont montés sur le récipient 2. Comme précédemment, il est entendu toutefois que le dispositif 1 de raccordement ou l'accessoire 3 ou la partie fonctionnelle 11 peut être considéré en soi, indépendamment du récipient 2, avant montage sur celui-ci.

Les deux états possibles sont un premier état qualifié de distal et un second état qualifié de proximal. Les qualificatifs de « premier » et « second » expriment l'idée qu'au départ, on se trouve dans l'état distal.

Dans le premier état distal, la partie proximale active 9 de l'accessoire 3, le cas échéant la partie extrême proximale 12 de la partie fonctionnelle 11, est totalement séparée, et de façon étanche, de l'intérieur 4 du récipient 2 par une tête 14 formant barrière, faisant partie du dispositif 1 de raccordement.

Dans le second état proximal, la partie proximale active 9 de l'accessoire 3, le cas échéant la partie extrême proximale 12 de la partie fonctionnelle 11, est en communication avec l'intérieur 4 et insérée dans le récipient 2. La tête 14 forme alors une protection de la partie proximale active 9 et, le cas échéant de la partie extrême proximale 12 de la partie fonctionnelle 11.

Dans le cas où l'accessoire 3 est une sonde 3a, le premier état distal est typiquement celui correspondant au stockage ou à la calibration, tandis que le second état proximal est typiquement celui de la mesure, pour le contenu du récipient 2, du paramètre correspondant à la sonde 3a employée.

Dans le cas où l'accessoire 3 est un tube 3b, le premier état distal est typiquement celui correspondant au stockage ou à l'inutilisation de la fonction remplissage ou vidange du tube 3b, tandis que le second état proximal est celui de la fonction remplissage ou vidange du tube 3b.

Le dispositif 1 de raccordement comprend un premier élément 15, un deuxième élément 16 et un troisième élément 17, distincts, assemblés ensemble et coopèrent fonctionnellement avec l'accessoire 3 et le cas échéant la partie fonctionnelle 11. Le dispositif 1 de raccordement comprend en outre une chambre 18.

Le premier élément 15 a plusieurs fonctions :
- Il assure la fixation rigide du dispositif 1 de raccordement au récipient 2.
- Il définit une voie de passage, entre l'intérieur 4 et l'extérieur 8 du récipient 2.
- Pour le deuxième élément 16, il sert de support et de guide en translation axiale le long de l'axe 1a, sur une course C donnée dont les deux fins de course, correspondent respectivement au premier état distal et au second état proximal.
- Il contribue à servir de guide pour le troisième élément 17.
- Il contribue à délimiter la chambre 18.

Le deuxième élément 16 a également plusieurs fonctions :
- Il sert de support au troisième élément 17, lequel est fixé rigidement sur lui.
- Il forme un élément mobile à coulissement axial le long de l'axe 1a sur la course C dont les deux fins de course, correspondent respectivement au premier état distal et au second état proximal.

Le troisième élément 17, auquel appartient la tête 14, a également plusieurs fonctions :
- Il sert de support à l'accessoire 3, et le cas échéant à la partie fonctionnelle 11, lequel (lesquels) est (sont) fixé(s) rigidement sur lui.
- Il forme un élément mobile à coulissement axial le long de l'axe 1a sur la course C dont les deux fins de course, correspondent respectivement au premier état distal et au second état proximal.
- Dans le premier état distal, il assure, grâce à la tête 14 la fermeture étanche de l'ouverture 7 en formant une barrière interdisant l'accès à l'intérieur 4 du récipient 2 et la communication entre l'accessoire 3 et, le cas échéant, à la partie fonctionnelle 11, avec l'intérieur 4 du récipient 2.
- Dans le second état proximal, il assure, grâce à la tête 14 une protection de la partie proximale active 9 de l'accessoire 3 et, le cas échéant, de la partie extrême proximale 12 de la partie fonctionnelle 11, surtout lorsque l'une et/ou l'autre est introduite à proprement parler dans l'intérieur 4 du récipient 2.
- Il contribue également à délimiter la chambre 18.

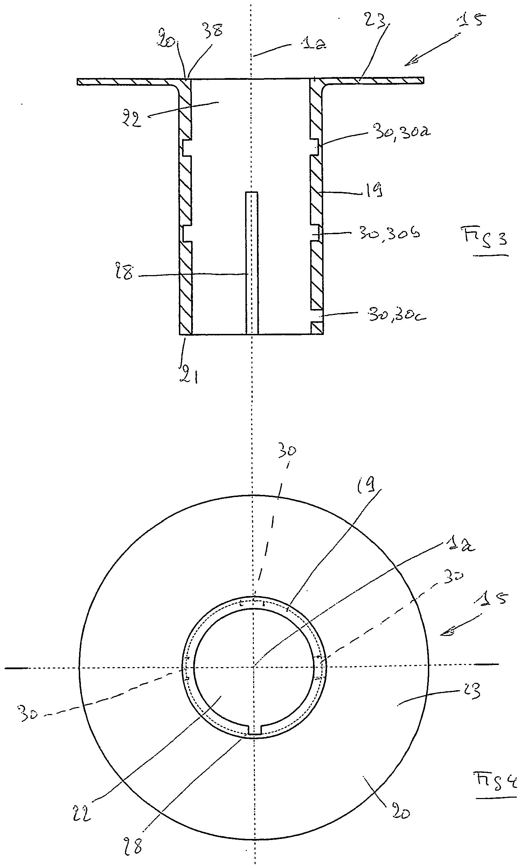

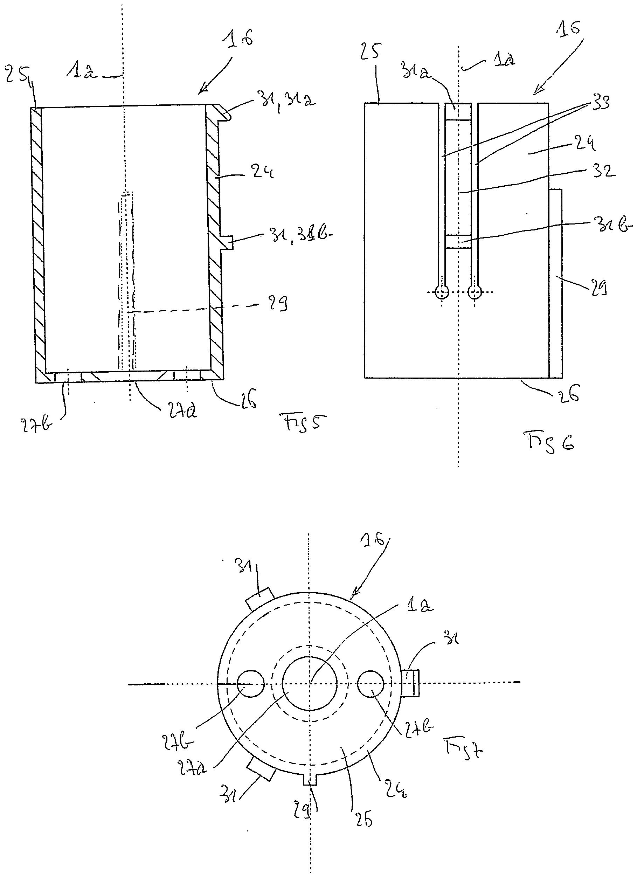

On décrit maintenant le premier élément 15 plus spécialement en référence aux figures 3 et 4 et le deuxième élément 16 plus spécialement en référence aux figures 5, 6 et 7.

Le premier élément 15, rigide et monobloc, comporte une paroi tubulaire 19, cylindrique à base carrée circulaire, s'étendant sur une certaine longueur axiale L1. Cette paroi tubulaire 19 est ouverte à ses deux extrémités, respectivement proximale 20 et distale 21, de manière à permettre le passage, vers l'extrémité proximale 20, du troisième élément 17, le cas échéant de la partie proximale active 9 de l'accessoire 3 et de la partie extrême proximale 12 de la partie fonctionnelle 11 et, vers l'extrémité distale 21, du deuxième élément 16, de l'accessoire 3 et de la partie fonctionnelle 11, notamment du côté de leurs parties distales 10, 13.

Le premier élément 15 et sa paroi tubulaire 19 forment une voie de passage 22 pour le contenu du récipient 2 depuis l'intérieur 4.

A l'extrémité proximale 20, le premier élément 15 comporte un rebord 23 de fixation au récipient 2 en forme de collerette annulaire transversale, dirigée latéralement vers l'extérieur. Le diamètre extérieur de la paroi tubulaire 19 est en correspondance avec le diamètre de l'ouverture 7, de manière que le premier élément 15 puisse s'ajuster dans l'ouverture 7. Le rebord 23, situé dans le prolongement de la paroi 6 et d'étendue radiale suffisante, est fixé à celle-ci autour de l'ouverture 7 de façon rigide et étanche, par exemple par soudage par ultrasons, collage ou d'une autre manière analogue.

Le premier élément 15 s'étend le long de l'axe 1a, vers l'extérieur 8 du récipient 2.

Le deuxième élément 16, rigide et monobloc, comporte une paroi tubulaire 24, cylindrique à base circulaire, s'étendant sur une certaine longueur axiale L2 qui dans la réalisation représentée sur les figures est un peu plus petite que la longueur L1.

Cette paroi tubulaire 24 est ouverte à son extrémité proximale 25 en étant ainsi apte à permettre le passage du troisième élément 17, de l'accessoire 3 et le cas échéant de la partie fonctionnelle 11. La paroi tubulaire 24 est ouverte à son extrémité distale 26 en étant ainsi apte à permettre le passage de l'accessoire 3 et le cas échéant de la partie fonctionnelle 11, notamment vers ou du côté de leurs parties distales 10, 13. Selon les réalisations, l'extrémité distale 26 est totalement ouverte ou elle comporte une paroi transversale pourvue d'une ouverture 27a pour la partie distale 10 de l'accessoire 3 et le cas échéant, d'une ouverture 27b pour la partie distale 13 de la partie fonctionnelle 11. Selon d'autres réalisations, il est prévu un plus grand nombre d'ouvertures, adapté au nombre d'accessoires 3 et de parties fonctionnelles 11.

Le diamètre extérieur de la paroi tubulaire 24 du deuxième élément 16 correspond au jeu nécessaire près au diamètre intérieur de la paroi tubulaire 19 du premier élément 15, de manière que le premier élément 15 forme pour le deuxième élément 16, un support et un guide en translation axiale le long de l'axe 1 a sur la course C.

La face intérieure de la paroi tubulaire 19 et la face extérieure de la paroi tubulaire 24 comportent une combinaison de rainure axiale 28 et de saillie radiale 29 vers l'extérieur. La saillie radiale 29 peut être logée ajustée dans, et coulisser le long de, la rainure axiale 28, sur une longueur axiale au moins égale à la course C. La combinaison rainure axiale 28 et saillie radiale 29 forme un détrompeur définissant et maintenant l'orientation angulaire relative entre le premier élément 15 et le deuxième élément 16.

Dans la réalisation représentée, la rainure 28 est ménagée sur le premier élément 15 et s'étend à partir de son extrémité distale 21. La saillie 29, radiale et axiale, est ménagée sur le deuxième élément 16 et s'étend à partir de son extrémité distale 24. Cette disposition constructive permet le montage du deuxième élément 16 sur le premier élément 15 par coulissement axial relatif du deuxième élément 16 dans le premier élément 15 à partir de son extrémité distale 21 vers son extrémité proximale 20.

La face intérieure de la paroi tubulaire 19 et la face extérieure de la paroi tubulaire 24 comportent une combinaison d'encoche(s) 30 et d'ergot(s) 31, escamotables, disposés radialement et aptes à coopérer. La combinaison encoche(s) 30 et d'ergot(s) 31 forme des moyens 30, 31, amovibles de blocage de fin de course de translation axiale le long de l'axe 1 a du deuxième élément 16 sur et par rapport au premier élément 15, sur la course C.

Pour permettre la coopération encoche 30/ergot 31, il est prévu qu'un ergot 31 est agencé de façon à être sollicité élastiquement radialement vers l'extérieur vers le fond d'une encoche 30 en regard afin de s'y loger. Pour permettre inversement l'autonomie encoche 30/ergot 31, il est prévu qu'un ergot 31 est agencé de façon à pouvoir être escamoté d'une encoche avec laquelle il coopérait, et ainsi s'effacer élastiquement radialement vers l'intérieur pour pouvoir ensuite glisser sur la face en regard de l'élément portant la ou les encoche(s) 30, une fois l'ergot 31 situé en dehors et à l'écart d'une encoche 30.

Ces moyens de blocage de fin de course de translation axiale à encoche(s) 30 et ergot(s) 31 peuvent faire l'objet de plusieurs réalisations.

Dans la réalisation la plus simple, il est prévu deux encoches 30 et un ergot 31 ou, inversement, une encoche 30 et deux ergots 31. Si le premier élément 15 reçoit un ergot 31, le deuxième élément 16 reçoit une encoche 30. Inversement, si le premier élément 15 reçoit une encoche 30, le deuxième élément 16 reçoit un ergot 31. Encoche(s) 30 et ergot(s) 31 sont positionnés angulairement de façon à pouvoir coopérer et ce compte tenu du détrompeur 28, 29. Ceux des encoches 30 ou des ergots 31 au nombre de deux sont, sur une même génératrice, écartés le long de l'axe 1a d'une distance C correspondant à la course de translation. Dans cette réalisation, chacune des deux fins de course de translation est définie par la coopération d'un ergot 31 dans une encoche 30.

Dans une autre réalisation, il est prévu que chaque encoche 30 et chaque ergot 31 est non pas unique mais double, triple, quadruple... dans un même plan transversal par rapport à l'axe 1a. Dans ce cas, ces encoches 30 et ergots 31 multiples sont préférentiellement également répartis angulairement autour de l'axe 1a. Dans cette réalisation, chacune des deux fins de course est définie par la coopération de plusieurs ergots 31 dans plusieurs encoches 30.

Dans une autre réalisation, il est prévu qu'une encoche 30 et/ou un ergot 31 n'est pas unique mais double, triple, quadruple... écartés judicieusement le long de l'axe 1a. Dans cette réalisation, chacune des deux fins de course est définie par la coopération de plusieurs ergots 31 dans plusieurs encoches 30.

Ces réalisations peuvent être combinées entre elles, chaque encoche 30 et chaque ergot 31 étant non pas unique mais double, triple, quadruple... dans un même plan transversal par rapport à l'axe 1 a et une encoche 30 et/ou un ergot 31 étant non pas unique mais double, triple, quadruple... le long de l'axe 1a. Une telle combinaison permet de diminuer le risque de désassemblage encoche 30 / ergot 31 et donc la préservation des positions de fin de course en l'absence de sollicitation extérieure intempestive.

Une telle combinaison correspond à la réalisation représentée sur les figures 3 à 7. Selon cette réalisation, les encoches 30 sont ménagées sur le premier élément 15 et les ergots 31 sont ménagés sur le deuxième élément 16. D'une part, chaque encoche 30 et chaque ergot 31 est triple avec un écartement angulaire de 120°. D'autre part, il est prévu trois telles encoches 30 écartées judicieusement le long de l'axe 1 a et deux tels ergots 31, également écartés le long de l'axe 1 a.

Selon cette réalisation, plus précisément, il est tout d'abord prévu sur la face intérieure de la paroi tubulaire 19 du premier élément 15, trois encoches proximales 30a, trois encoches intermédiaires 30b et trois encoches distales 30c. Les trois encoches proximales 30a sont situées vers mais écartées de l'extrémité proximale 20. Les trois encoches distales 30c sont situées à proximité immédiate de l'extrémité distale 21. Les trois encoches intermédiaires 30b sont également écartées axialement des encoches proximales 30a et des encoches distales 30c. Il est prévu ensuite sur la face extérieure de la paroi tubulaire 24 du deuxième élément 16, trois ergots proximaux 31 a et trois ergots distaux 31 b. Les trois ergots proximaux 31 a sont situés à proximité immédiate de l'extrémité proximale 22. Les trois ergots distaux 31 b sont sensiblement également écartés axialement des extrémités proximale 23 et distale 24.

Dans le premier état distal, les trois ergots proximaux 31 a coopèrent avec les trois encoches intermédiaires 30b et les trois ergots distaux 31 b coopèrent avec les trois encoches distales 30c.

Dans le second état proximal, les trois ergots proximaux 31 a coopèrent avec les trois encoches proximales 30a et les trois ergots distaux 31 b coopèrent avec les trois encoches intermédiaires 30b.

En conséquence, dans le premier état distal, le deuxième élément 16 est latéralement en regard du premier élément 15, sans être en regard d'une partie du premier élément 15 attenante à son extrémité proximale 20, une partie du deuxième élément 16 saillant de l'extrémité distale 21 du premier élément 15.

Dans le second état proximal, le deuxième élément 16 est latéralement tout entier en regard du premier élément 15 vers son extrémité distale 21, sans être en regard d'une partie plus courte axialement du premier élément 15 attenante à son extrémité proximale 20.

En ce qui concerne la coopération/autonomie encoche/ergot, il est prévu, dans la réalisation représentée, que deux ergots 31 a, 31 b, écartés le long de l'axe 1a sont portés par une patte 32, de direction axiale, élastiquement déformable dans son ensemble, formée par une partie de la paroi tubulaire 24 attenante à une extrémité, distale dans la réalisation représentée, mais séparée ailleurs du reste de la paroi tubulaire 24 par une fente 33 (avec ou sans enlèvement de matière). Dans la réalisation représentée, l'extrémité proximale de la patte 24 est confondue avec l'extrémité proximale 25 du deuxième élément 16. Quant à son extrémité distale, elle est située, le long de l'axe 1 a, entre l'ergot distal 31 b et l'extrémité distale 26 du deuxième élément 16. Par cette disposition constructive, une même patte 32 peut porter les deux ergots 31 a, 31 b, l'ergot distal 31 b étant suffisamment éloigné du raccordement entre la patte 32 et la paroi tubulaire 24 pour pouvoir être escamoté par suite de la déformation de la patte.

Avantageusement, les premier et deuxième éléments 15 et 16 sont réalisés en polyéthylène ou en polypropylène.

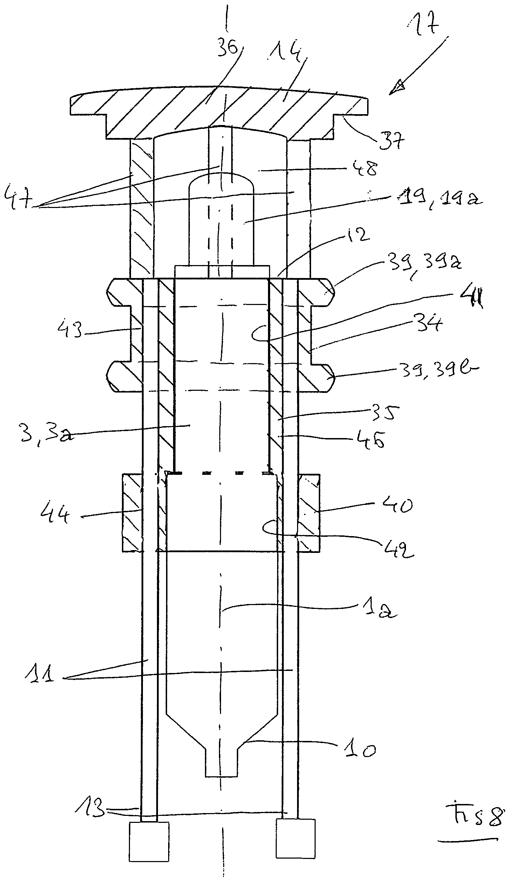

On décrit maintenant le troisième élément 17 plus spécialement en référence à la figure 8.

Le troisième élément 17, en plusieurs pièces, de révolution autour de l'axe 1 a, comporte la tête 14, une base 34 et un corps 35, formant un ensemble rigide.

La tête 14 a deux fonctions :
- Dans le premier état distal, la tête 14 ferme de façon étanche l'ouverture 7 et le premier élément 15, en formant une barrière interdisant l'accès à l'intérieur 4 du récipient 2 et la communication entre l'accessoire 3 et, le cas échéant, à la partie fonctionnelle 11, avec l'intérieur 4 du récipient 2.
- Dans le second état proximal, la tête 14 contribue à la protection de la partie proximale active 9 et, le cas échéant, de la partie extrême proximale 12, surtout lorsque l'une et/ou l'autre est introduite à proprement parler dans l'intérieur 4 du récipient 2, en ayant franchi l'ouverture 7. En effet, il se peut, par exemple, qu'il y ait dans le contenu des agglomérats ou que l'on ait introduit dans le récipient 2 un organe (tube, arbre, hélice, distributeur...) susceptibles dans un cas comme dans l'autre de détériorer la partie proximale active 9, le cas échéant la partie extrême proximale 12, introduite dans l'intérieur 4 du récipient 2.

La tête 14 se présente sous la forme générale d'une paroi transversale 36, pleine, formant une sorte de couvercle ou clapet, apte à coopérer avec l'ouverture 7 pour la fermer de façon étanche lorsque nécessaire et à en être écartée par coulissement axial pour l'ouvrir.

Le chant périphérique de la tête 14 forme un décrochement 37 apte à coopérer avec, et à épouser de façon étanche, le siège d'appui et de retenue formé par l'extrémité proximale 20 et la partie attenante 38 du rebord 23.

La tête 14 présente une déformabilité élastique d'ensemble permettant, d'une part, d'assurer la coopération étanche avec le siège d'appui et de retenue, et, d'autre part, d'être déformée dans le sens d'une diminution de son diamètre extérieur, de manière à pouvoir - pour le montage - être introduite dans le premier élément 15 par son extrémité distale 21.

Dans le premier état distal, la tête 14 repose sur le siège que forme le premier élément 15 en fermant l'ouverture 7 de façon étanche. Elle est alors située sinon dans, du moins proche du, plan de l'ouverture 7 et de la paroi 6. Dans le second état proximal, la tête 14 est introduite dans l'intérieur 4 du récipient 2, en franchissant l'ouverture 7 de la paroi 6 pour aller au-delà de cette dernière.

La base 34 a plusieurs fonctions :
- Elle sert de support à l'accessoire 3, et éventuellement à la partie fonctionnelle 11, lequel (lesquels) est (sont) fixé(s) rigidement sur elle.
- En combinaison avec le premier élément 15, elle assure le guidage de coulissement, avec étanchéité, du troisième élément 17 sur et par rapport au premier élément 15.
- Elle contribue à délimiter la chambre 18.

La base 34 comporte, en premier lieu, dans sa partie extrême proximale une première bague 39, apte à pouvoir coulisser axialement, avec étanchéité, par sa face extérieure, sur et le long de la face intérieure de la paroi tubulaire 19 du premier élément 15. A cet effet, la première bague de guidage 39 a un diamètre nominal extérieur correspondant au diamètre intérieur de la paroi tubulaire 19, de manière qu'il y ait contact coulissant et étanchéité entre la face extérieure de la première bague et la face intérieure de la paroi tubulaire 19 du premier élément 15

Dans la réalisation représentée, il est prévu deux premières bagues 39 a et 39 b espacées l'une de l'autre le long de l'axe 1a, ce qui permet de combiner à la fois un bon guidage par une portée suffisante, un bon coulissement et une bonne étanchéité.

La base 34 comporte, en second lieu, à sa partie extrême distale une seconde bague 40, apte à être fixée rigidement par sa face extérieure, sur et contre la face intérieure de la paroi tubulaire 24 du deuxième élément 16, dans ou au voisinage de sa partie médiane entre ses extrémités proximale 25 et distale 26. Ainsi, la première bague 39 est située à l'extérieur du deuxième élément 16, mais à proximité immédiate de son extrémité proximale 25. La seconde bague 40 est donc dimensionnée et positionnée à l'effet d'obtenir ces résultats.

Les faces intérieures 41 et 42, respectivement, de la première bague 39 et la seconde bague 40 sont aptes à permettre la fixation rigide, avec une étanchéité globale, de l'accessoire 3 par sa face extérieure. La position axiale de l'accessoire 3 par rapport à la base 34 est telle que la partie proximale active 9 saille de façon proximale de la première bague 39 ou affleure son extrémité proximale. Le premier cas est bien adapté lorsque l'accessoire 3 est une sonde 3a tandis que le second cas est bien adapté lorsque l'accessoire 3 est un tube d'amenée ou de vidange 3b. Dans tous les cas, les faces intérieures 41 et 42 sont dimensionnées à l'effet de permettre cette fixation rigide et globalement étanche. Cette fixation rigide est réalisée par exemple par collage.

Le cas échéant, la première bague 39 et la seconde bague 40 comportent au moins deux canaux en alignement 43 et 44, aptes à permettre le montage à l'intérieur ainsi que la fixation rigide, avec étanchéité, de la partie fonctionnelle 11 placée à côté ou à proximité de l'accessoire 3 en étant excentrée par rapport à l'axe 1a. La position axiale de la partie fonctionnelle 11 par rapport à la base 34 est telle que la partie extrême proximale 12 affleure l'extrémité proximale de la première bague 39 ou saille de façon proximale de celle-ci. Le premier cas est bien adapté lorsque la partie fonctionnelle 11 est un tube d'amenée ou de vidange, tandis que le second cas est bien adapté lorsque la partie fonctionnelle 11 est par exemple un organe de mesure. La fixation rigide de la partie fonctionnelle 11 est réalisée par exemple par collage.

Le corps 35 comporte une partie de liaison 46 reliant la première et la seconde bagues 39 et 40 et comportant un alésage 45 traversant, axial, dans lequel l'accessoire 3 est inséré. Le cas échéant, l'accessoire 3 est également fixé à la face de cet alésage 45, donc à la partie de liaison 46.

Dans la réalisation représentée, cette partie de liaison 46 présente un diamètre extérieur plus faible que le diamètre extérieur des première et seconde bagues 39 et 40. Cette disposition permet à la partie fonctionnelle 11 si elle existe d'être placée à l'extérieur de la partie de liaison 46.

Le cas échéant, la partie de liaison 46 comporte au moins un canal ou une encoche en alignement avec les canaux 43 et 44.

Le corps 35 comporte également un ou des organes porteurs 47, reliant rigidement la tête 14 et la base 34, à savoir l'extrémité proximale de la première bague 39. Ce (ou ces) organes porteur(s) 47 est (sont) assez rigide(s) pour assurer un maintien efficace de la tête 14. D'autre part, ce (ou ces) organe(s) porteur(s) 47 est (sont) agencé(s) pour permettre un passage à travers du contenu du récipient 2 et/ou d'un fluide amené ou vidangé par une partie fonctionnelle prévue à cet effet, par exemple pour la calibration de la sonde 3a.

Les organes porteurs 47 peuvent se présenter sous forme de tiges s'étendant axialement ménageant entre elles des passages 48 ou sous forme d'un manchon pourvu de tels passages 48.

Dans la réalisation plus spécialement considérée, le troisième élément 17, à l'exception des organes porteurs 47, est réalisé en une seule pièce par surmoulage, dans un matériau assurant une bonne étanchéité et présentant une certaine déformabilité élastique comme déjà indiqué pour la tête 14, et une certaine qualité à glisser, tel que le silicone. En ce qui concerne les organes porteurs 47, ils sont avantageusement réalisés en acier inoxydable, ou tout autre matériau présentant la rigidité souhaitée. Dans d'autres réalisations, le troisième élément est réalisé en plusieurs pièces autrement assemblées.

Le troisième élément 17 s'étend sur une certaine longueur axiale L3, légèrement plus petite que L1.
L'accessoire 3 s'étend sur une certaine longueur axiale L4, en l'espèce un peu plus grande que L1 et L3.

La longueur axiale des organes porteurs 47 est adaptée pour permettre le logement de la partie proximale active 9, lorsqu'elle saille de l'extrémité proximale de la première bague 39, et, dans le second état proximal, le passage sans entrave du contenu du récipient 2 vers la chambre 18 où se trouve une sonde 3a, ou du fluide provenant du ou allant vers le tube 3b.

Dans le premier état distal, le troisième élément 17 est tout entier logé dans le premier élément 15, à l'exception de la tête 14. Dans le second état proximal, la tête 14 et les organes porteurs 47 du troisième élément 17 sont situés hors du premier élément 15, la première bague 39 étant située dans l'ouverture 7. Dans ce second état proximal, la première bague 39, la seconde bague 40 et la partie de liaison 46 sont entièrement logées dans le premier élément 15, la seconde bague 40 étant située vers l'extrémité distale 21 du premier élément 15.

La position relative du troisième élément 17 par rapport au deuxième 16 est fixe. La seconde bague 40 et la partie de liaison 46 sont tout entier situées dans le deuxième élément 16. La première bague 39, les organes porteurs 47 et la tête 14 sont tout entier situés à l'extérieur du deuxième élément 16.

Dans le premier état distal, la chambre 18 est fermée de façon étanche et elle est délimitée par le premier élément 15 et le troisième élément 17, si l'on fait abstraction de la partie proximale active 9 de l'accessoire 3 qui, logée à l'intérieur (cas de la partie proximale active 9a) ou placée en périphérie (cas de la partie proximale active 9b) de la chambre 18, contribue d'une certaine manière à la délimiter.

Plus précisément, la chambre 18 est limitée en premier lieu, à ses deux parties extrêmes axiales, d'une part, par la face transversale distale de la paroi 36 formant la tête 14, laquelle ferme l'ouverture 7 et, d'autre part, par la face transversale d'extrémité proximale de la première bague 39. La chambre 18 est limitée en second lieu, à sa périphérie, par la face interne de la paroi tubulaire 19 du premier élément 15.

L'étanchéité de la chambre 18, dans le premier état distal, est assurée par l'étanchéité prévue entre la tête 14 et son siège appartenant au premier élément 15, entre la première bague 39 et la face interne de la paroi tubulaire 19, et entre l'accessoire 3 et le troisième élément 17, le cas échéant entre la première bague 39 et la partie fonctionnelle 11.

La chambre 18 est telle que la partie proximale active 9 y est soit logée à l'intérieur (cas de la partie proximale active 9a d'une sonde 3a), soit placée en périphérie (cas de la partie proximale active 9b d'un tube 3b), de sorte que la partie proximale active 9 est en contact ou en communication avec la chambre 18 ou le fluide qu'elle contient.

Dans le second état proximal, la chambre 18 est ouverte, la tête 14 étant écartée de l'ouverture 17, alors ouverte. Ailleurs, l'étanchéité précédemment mentionnée est maintenue.

Selon les applications et les besoins, la chambre 18 peut être stérile ou non stérile humide ou sèche, en fonction de la sonde 3a. Par exemple, une sonde pH requiert une ambiance humide. Cette ambiance humide est obtenue par la présence, dans la chambre 18 d'un liquide *ad hoc,* injecté au moment du stockage et désigné pour cette raison « liquide de stockage ».

Dans le cas où, au contraire, la sonde 3a requiert non pas une ambiance humide mais une ambiance sèche, comme dans le cas où l'accessoire 3 est un tube, il n'est pas prévu un tel liquide de stockage.

La chambre 18 peut, après montage du dispositif 1 de raccordement pourvu de l'accessoire 3 et vidange de l'air (ou du gaz) qui s'y trouve, remplir comme fonction d'y loger à l'intérieur ou d'y placer en périphérie la partie proximale active 9, comme il a été décrit.

La chambre 18 peut également une ou plusieurs autres fonctions, et cela en liaison avec les usages souhaités.

Une fonction possible de la chambre 18 est d'y mettre un liquide de stockage, comme il a été exposé, tel que du chlorure de potassium ou une solution de chlorure de potassium. Puis, il est possible de procéder à une stérilisation, par exemple une stérilisation à rayons gamma.

Une autre fonction possible de la chambre 18, dans le cas où l'accessoire 3 est une sonde 3a, est de procéder à une calibration de celle-ci. A cet effet, on met dans la chambre 18 un liquide de calibration.

Une autre fonction possible de la chambre 18, dans le cas où l'accessoire 3 est un tube 3b, est de constituer une chambre fermée de façon étanche dans lequel est placé le tube 3b en attente d'utilisation.

Le procédé de montage du dispositif 1 de raccordement et de l'accessoire 3, le cas échéant de la partie fonctionnelle 11, sur un récipient 2 est réalisé comme suit sur le site de fabrication, notamment dans des conditions « salle blanche ».

On part d'une situation où l'on dispose d'un récipient 2, vide de contenu, de l'accessoire 3, le cas échéant de la partie fonctionnelle 11, enfin des éléments 15, 16 et 17 constitutifs du dispositif 1 de raccordement.

La description du montage et de la fabrication est faite dans le cas d'un récipient 2 souple et à usage unique tel qu'une poche dite 2D ou une poche pliable dite 3D.

D'une part, dans une étape préliminaire, on fixe rigidement le premier élément 15 sur le récipient à l'endroit de l'ouverture 17, par soudage thermique ou d'une autre manière analogue.

D'autre part, dans une autre étape préliminaire, on insère l'accessoire 3 dans le troisième élément 17, on le positionne axialement de façon convenable et on le fixe rigidement à celui-ci à l'endroit des première et seconde bagues 39 et 40, le cas échéant dans l'alésage 45. Le cas échéant, également, on insère, positionne et fixe la partie fonctionnelle 11. Cette insertion est réalisée par coulissement axial relatif, à partir de l'extrémité distale de la seconde bague 40 et vers la tête 14. Par cette étape, on commence à constituer la chambre 18.

Puis, dans une deuxième étape ultérieure à cette autre étape préliminaire, on monte l'ensemble troisième élément 17 + accessoire 3 + éventuellement partie fonctionnelle 11, sur et dans le deuxième élément 16. Ce montage est réalisé par coulissement axial relatif du dit ensemble 17, 3 dans le deuxième élément 16 à partir de son extrémité proximale 25 et vers son extrémité distale 26. Il convient, bien entendu, que faire en sorte que la partie distale 10 de l'accessoire 3 et les moyens tels que conducteur électrique, tube... qui y sont associés, et le cas échéant, la partie distale 13 de la partie fonctionnelle 11, traverse l'extrémité distale 24 totalement ouverte du deuxième élément 16 ou les ouvertures 27a et 27b prévues à cet effet.

Puis, dans une troisième étape ultérieure à cette deuxième étape, on monte l'ensemble deuxième élément 16 + troisième élément 17 + accessoire 3 + éventuellement partie fonctionnelle 11, sur et dans le premier élément 15. Ce montage est réalisé par coulissement axial relatif du dit ensemble 16, 17, 3 dans le premier élément 15 à partir de son extrémité distale 21 vers son extrémité proximale 20, dans la position angulaire définie par le détrompeur 28, 29. L'introduction de la tête 14 dans l'extrémité distale ouverte est rendue possible grâce à lé déformabilité élastique de la tête. Le coulissement est poursuivi jusqu'à coopération des encoche(s) 30 et ergot(s) 31 correspondant au premier état distal. On arrive alors en bout de course, sauf action spécifique en vue de désengager les encoche(s) 30 et ergot(s) 31, leur coopération restant assurée du fait de la ou des pattes élastiques 32. Par cette étape, on finit de constituer la chambre 18, alors fermée de façon étanche.

L'ensemble dispositif 1 de raccordement + accessoire 3 + éventuellement partie fonctionnelle 11 est alors en place, dans le premier état distal, le récipient 2 étant vide de contenu. La chambre 18 est alors vide de contenu autre que l'atmosphère ambiante dans laquelle le dispositif 1 de raccordement a été monté sur le récipient 2.

Lorsque cela est souhaité, à l'aide d'une seringue que l'on connecte soit sur l'embout de la partie extrême distale 13 de la partie fonctionnelle 11, dans le cas où l'accessoire 3 est une sonde 3a, soit sur l'extrémité distale 10 de l'accessoire 3, dans le cas où l'accessoire 3 est un tube 3b, on aspire l'air (ou le gaz) se trouvant dans la chambre 18 et on la vide de celui-ci. Ensuite, on déconnecte et on retire la seringue.

Alors, on peut, à l'aide d'une seringue que l'on connecte comme précédemment, injecter dans la chambre 18 un liquide de stockage, tel que du chlorure de potassium ou une solution de chlorure de potassium. Ensuite, on déconnecte et on retire la seringue. Une telle réalisation s'impose lorsque l'acessoire 3 est une sonde 3a qui doit être maintenue en milieu humide, comme tel est le cas d'une sonde pH. Si, au contraire, l'accessoire 3 est une sonde 3a qui doit être maintenu en milieu sec, comme tel est le cas d'une sonde colorimétrie, il n'est pas prévu d'injecter de liquide de stockage.

Puis, il est possible de procéder à une stérilisation de l'ensemble récipient 2 + dispositif 1 de raccordement + accessoire 3 +, le cas échéant la partie fonctionnelle 11, par exemple une stérilisation à rayons gamma.

Les différentes étapes du procédé de montage du dispositif 1 de raccordement et de l'accessoire 3, le cas échéant de la partie fonctionnelle 11, sur un récipient 2 qui viennent d'être décrites sont réalisées le plus souvent les unes à la suite des autres sur un site de fabrication des conteneurs fonctionnels 5.

Le second ensemble récipient 2 plié + dispositif 1 de raccordement + accessoire 3 +, le cas échéant la partie fonctionnelle 11, forme un ensemble plat qui peut alors être conditionné, par exemple dans une première poche en matière plastique elle-même placée dans un carton d'emballage.

Le second ensemble récipient 2 + dispositif 1 de raccordement + accessoire 3 +, le cas échéant la partie fonctionnelle 11, peut alors être stocké et transporté jusqu'à utilisation, celle-ci intervenant sur un site d'utilisation généralement différent du site de fabrication des conteneurs fonctionnels 5 et un certain temps après que la fabrication est intervenue, l'asepsie étant préservée.

Le procédé de mise en oeuvre du second ensemble récipient 2 + dispositif 1 de raccordement + accessoire 3 +, le cas échéant la partie fonctionnelle 11, + les éventuels moyens rigides de maintien extérieur du récipient 2 si celui-ci est souple, sur le site d'utilisation, est réalisé comme suit, une fois l'ensemble déconditionné du carton d'emballage et de la poche ayant servi au conditionnement.

Ce procédé de mise en oeuvre comporte des étapes opératoires liées au récipient 2, en soi, indépendamment du fait qu'il est raccordé au dispositif 1 de raccordement et à l'accessoire 3 et le cas échéant à la partie fonctionnelle 11. Ces étapes opératoires comprennent les suivantes :
- Placer le récipient 2, s'il s'agit d'un récipient 2 souple, dans les moyens rigides de maintien extérieur, pour autant que cela soit nécessaire (cas d'une poche 3D).
- Emplir le récipient 2 avec le contenu.
- Réaliser tout traitement approprié sur le contenu du récipient 2, tel que réaction, mélange, aération, addition d'autres composants...
- Eventuellement, transporter ou stocker de façon temporaire le récipient 2 et son contenu.
- Vidanger le récipient 2 de son contenu, afin d'utiliser celui-ci.

Ces étapes opératoires sont à la portée de l'homme du métier et n'ont pas à être décrites.

Le procédé de mise en oeuvre comporte par ailleurs des étapes opératoires liées spécifiquement au dispositif 1 de raccordement, à l'accessoire 3 et le cas échéant à la partie fonctionnelle 11. Ces étapes opératoires sont les suivantes :
- Dans une étape préliminaire, vider de la chambre 18 le liquide de stockage pour autant qu'un tel liquide de stockage y ait été mis lors de la fabrication et dans ce cas, le cas échéant, rincer la chambre 18, ou sinon, ne pas faire cette étape préliminaire.
- Faire passer tout d'abord l'ensemble dispositif 1 de raccordement + accessoire 3 +, le cas échéant la partie fonctionnelle 11, du premier état distal dans lequel il se trouvait à l'issue de la fabrication, au second état proximal.
- Puis, mettre en oeuvre l'accessoire 3.

Dans une réalisation, le procédé comporte également une opération supplémentaire de réglage, de contrôle ou de calibration de l'accessoire 3.

Dans une réalisation particulière correspondant au cas où l'accessoire 3 est une sonde 3a qui doit être stockée en milieu humide (par exemple sonde pH) et qui nécessite une telle opération supplémentaire de réglage, de contrôle ou de calibration de l'accessoire 3, la séquence est la suivante :
- Vider de la chambre 18 le liquide de stockage qui y a été mis lors de la fabrication, le cas échéant rincer la chambre 18.
- Réaliser une opération de réglage, de contrôle ou de calibration de la sonde 3a.
- Placer le récipient 2, s'il s'agit d'un récipient 2 souple, dans les moyens rigides de maintien extérieur.
- Emplir le récipient 2 avec le contenu.
- Faire passer l'ensemble dispositif 1 de raccordement + sonde 3a + tube 11, du premier état distal dans lequel il se trouvait à l'issue de la fabrication, au second état proximal.
- Réaliser tout traitement approprié sur le contenu du récipient 2, tel que réaction, mélange, aération, addition d'autres composants...
- Mettre en oeuvre la sonde 3a, en continuant de réaliser le traitement approprié sur le contenu.
- Réaliser une autre opération de réglage, de contrôle ou de calibration de la sonde 3a.
- Eventuellement, transporter ou stocker de façon temporaire le récipient 2 et son contenu.
- *In fine,* vidanger le récipient 2 de son contenu, afin d'utiliser celui-ci.

Cette séquence d'étapes n'est toutefois pas exclusive d'autres.

On décrit maintenant les étapes opératoires liées spécifiquement au dispositif 1 de raccordement et à l'accessoire 3, dans la réalisation particulière où l'accessoire 3 est une sonde 3a qui nécessite une opération supplémentaire de réglage, de contrôle ou de calibration de l'accessoire 3, et où la partie fonctionnelle 11 est un tube d'amenée et de vidange. Les autres réalisations de ces étapes peuvent être déduites de cette réalisation particulière.

En ce qui concerne l'étape consistant à vider de la chambre 18 le liquide de stockage qui y a été mis lors de la fabrication, on procède comme suit.

A l'aide d'une seringue que l'on connecte sur l'embout de la partie extrême distale 13 du tube 11, on aspire le liquide de stockage se trouvant dans la chambre 18 et on la vide de celui-ci. Ensuite, on déconnecte et on retire la seringue.

En ce qui concerne l'étape consistant à faire passer l'ensemble dispositif 1 de raccordement + sonde 3a + tube 11, du premier état distal dans lequel il se trouvait à l'issue de la fabrication, au second état proximal, on procède comme suit.

On fait coulisser axialement l'ensemble deuxième élément 16 + troisième élément 17 + accessoire 3 + tube 11, sur la course C, dans le sens proximal, c'est-à-dire vers le récipient 2, jusqu'à coopération des encoche(s) 30 et ergot(s) 31 correspondant au second état proximal. On arrive alors en bout de course.

Le passage de la coopération des encoche(s) 30 et ergot(s) 31 correspondant au premier état distal à leur coopération correspondant au second état proximal est rendu possible du fait de la flexibilité de la ou des pattes 32.

Pour permettre le mouvement de retrait de désengagement d'un ergot 31 d'une encoche, on utilise un outil (non représenté) apte à déplacer l'ergot 31 à l'encontre de la patte 32.

Lorsque, par le mouvement unique qui vient d'être décrit, on passe du premier état distal au second état proximal, on ouvre la voie de passage 22 qui était jusqu'alors fermée de façon étanche par la tête 14 et on met en communication la partie active proximale 9a de la sonde 3a avec l'intérieur 4, cette partie active proximale 9a étant ainsi insérée dans le récipient 2, comme il a été indiqué plus haut. La chambre 18 qui était jusqu'ici fermée est ouverte et si le récipient 2 contient un contenu dépassant le niveau de l'ouverture 7, ce contenu peut remplir la chambre 18. La partie active proximale 9a est également en contact ou en communication avec ce contenu.

Dans le second état proximal, la tête 14 remplit sa fonction de protection, tandis que la première bague 39 assure l'étanchéité du récipient 2.

Le dispositif 1 de raccordement est réversible en ce sens qu'il est possible de le faire repasser depuis le second état proximal au premier état distal par un mouvement opposé à celui précédemment décrit pour passer du premier état distal au second état proximal.

Cette opération permet de capturer du contenu du récipient 2 dans la chambre 18, qui a été fermée.

En ce qui concerne l'étape proprement dite de mise en oeuvre de la sonde 3a, c'est-à-dire de mesure par celle-ci du paramètre auquel elle est adaptée, elle est à la portée de l'homme du métier et n'a pas à être décrite.

En ce qui concerne l'étape consistant en l'opération supplémentaire de réglage, de contrôle ou de calibration de la sonde 3a, on procède comme suit.

Cette étape, désignée maintenant conventionnellement « calibration », peut être réalisée de façon unique ou au contraire plusieurs fois, et elle peut l'être à différents moments et à plusieurs fins.

Dans une réalisation, il est procédé à deux calibrations :
- Une calibration initiale, avant que le récipient 2 ne soit empli de son contenu. Cette calibration a pour but de s'assurer que la sonde 3a est correctement réglée et, à défaut, de la régler de façon appropriée.
- Une calibration finale, après que le récipient 2 a été vidangé de son contenu. Cette calibration est faite à des fins de contrôle que la sonde 3a ne s'est pas déréglée durant le process.

Le cas échéant, on peut envisager une (voire plusieurs) calibration(s) intermédiaire(s) après que le récipient 2 a été empli (totalement ou partiellement) de son contenu et avant qu'il a été vidangé de son contenu. Une telle calibration a pour but de s'assurer, en cours de process, que la sonde 3a est correctement réglée et, à défaut, de la régler de façon appropriée.

La calibration est réalisée au moyen de la chambre 18, dont c'est l'une des fonctions.

Dans tous les cas, la calibration est réalisée alors que l'ensemble dispositif 1 de raccordement + sonde 3a + tube 11 est dans le premier état distal et que la chambre 18 est fermée de façon étanche. Soit l'ensemble dispositif 1 de raccordement + sonde 3a + tube 11 est dans le premier état distal pour la raison qu'il s'y trouvait antérieurement, par exemple à l'issue de la fabrication, soit cet ensemble est amené dans le premier état distal à partir du second état proximal dans lequel il se trouvait antérieurement. Cela est possible car le dispositif 1 de raccordement est réversible.

La présence de la chambre 18 permet une calibration en ligne, en ce sens qu'elle peut être réalisée alors que le dispositif 1, la sonde 3a et le tube 11 ont déjà été raccordé au récipient 2.

Etant, comme indiqué, dans le premier état distal, on injecte d'abord une solution pH de calibration dans la chambre 18 fermée de façon étanche, à l'aide d'une seringue sans aiguille, via le tube 11 au moyen de l'embout de connexion de la partie distale 13. Une fois l'injection terminée, la seringue est retirée de l'embout de connexion.

Puis, la solution pH de calibration remplissant ainsi la chambre 18 toujours fermée de façon étanche, et la partie proximale active 9a étant en contact avec la solution pH de calibration, on calibre la sonde 3a si cela est nécessaire et comme cela est requis.

Puis, la chambre 18 toujours fermée de façon étanche, on vidange de la solution pH de calibration à l'aide d'une seringue sans aiguille via un tube 11 de vidange, au moyen de l'embout de connexion à la partie distale 13. Une fois la vidange terminée, la seringue est retirée de l'embout de connexion.

On connecte alors une seringue de rinçage contenant un produit de rinçage tel que de l'eau stérile sur le tube 11 par l'intermédiaire de l'embout de connexion. On injecte le produit de rinçage dans la chambre 18, puis on le vidange.

L'opération de calibration ayant été ainsi effectuée, on peut ouvrir la chambre 18 en passant du premier état distal au second état proximal. La sonde 3a peut alors mesurer le paramètre pour lequel elle est destinée dans le récipient 2, et plus spécialement son contenu.

## Revendications

1. Dispositif (1) de raccordement à un récipient (2) d'un accessoire (3) dont la partie proximale active (9) est apte à être mis en communication avec l'intérieur (4) du récipient (2) par l'intermédiaire d'une ouverture (7) du récipient (2), le dispositif comprenant un premier élément (15), un deuxième élément (16), un troisième élément (17) et une chambre (18), dans lequel :
■ le premier élément (15) a pour fonctions :
○ d'assurer la fixation rigide du dispositif (1) de raccordement au récipient (2),
○ de définir une voie de passage (22), entre l'intérieur (4) et l'extérieur (8) du récipient (2),
○ de servir de support pour le deuxième élément (16) et de guide en translation axiale sur une course axiale C dont les deux fins de course correspondent à un premier état distal et à un second état proximal,
○ de contribuer à servir de guide pour le troisième élément (17),
○ de contribuer à délimiter la chambre (18),
■ le deuxième élément (16) a pour fonctions :
○ de servir de support au troisième élément (17), lequel est fixé rigidement sur lui,
○ de former un élément mobile à coulissement axial sur la course C dont les deux fins de course correspondent au premier état distal et au second état proximal,
■ le troisième élément (17), auquel appartient une tête (14) a pour fonctions :
○ de servir de support à l'accessoire (3), lequel est fixé rigidement sur lui,
○ de former un élément mobile à coulissement axial sur la course C dont les deux fins de course, correspondent au premier état distal et au second état proximal,
○ dans le premier état distal, d'assurer, grâce à la tête (14), la fermeture étanche de l'ouverture (7),
○ dans le second état proximal, d'assurer, grâce à la tête (14), une protection de la partie proximale active (9),
○ de contribuer à délimiter la chambre (18),
■ la chambre (18) est dans le premier état distal, fermée de façon étanche et, dans le second état proximal, en communication avec l'intérieur (4) et ayant pour fonction d'y loger à l'intérieur ou d'y placer en périphérie la partie proximale active (9), et, le cas échéant,
○ d'y mettre un liquide de stockage,
○ d'y mettre un liquide de réglage, contrôle ou calibration de l'accessoire (3) ou de constituer une chambre (18) fermée de façon étanche dans lequel est placé l'accessoire (3) en attente d'utilisation.

2. Dispositif (1) de raccordement selon la revendication 1, **caractérisé par le fait que** troisième élément (17) comporte outre la tête (14), une base (34) et un corps (35),
■ la tête (14) ayant pour fonctions :
○ dans le premier état distal, de fermer de façon étanche le premier élément (15),
○ dans le second état proximal, de contribuer à la protection de la partie proximale active (9),
■ la base (34) ayant pour fonctions :
○ de servir de support à l'accessoire (3), lequel est fixé rigidement sur elle,
○ d'assurer le guidage de coulissement, avec étanchéité, du troisième élément (17) sur et par rapport au premier élément (15),
○ de contribuer à délimiter la chambre (18),
■ le corps (35) comportant une partie de liaison (46) pourvue d'un alésage (45) dans lequel l'accessoire (3) est inséré et au moins un organe porteur (47), reliant rigidement la tête (14) et la base (34).

3. Dispositif (1) de raccordement selon la revendication 2, **caractérisé par le fait que** la base (34) comporte :
■ dans sa partie extrême proximale, au moins une première bague (39), apte à pouvoir coulisser axialement, avec étanchéité, par sa face extérieure, sur et le long de la face intérieure de la paroi tubulaire (19),
■ à sa partie extrême distale, une seconde bague (40), apte à être fixée rigidement par sa face extérieure, sur et contre la face intérieure de la paroi tubulaire (24) du deuxième élément (16),
■ des faces intérieures (41, 42) de la bague (39, 40), aptes à permettre la fixation rigide, avec étanchéité, de l'accessoire (3).

4. Dispositif (1) de raccordement selon la revendication 3, **caractérisé par le fait que** la position relative du troisième élément (17) par rapport au deuxième élément (16) est fixe, la seconde bague (40) et la partie de liaison (46) étant situées dans le deuxième élément (16), la première bague (39), les organes porteurs (47) et la tête (14) étant situés à l'extérieur du deuxième élément (16).

5. Dispositif (1) de raccordement selon l'une quelconque des revendications 1 à 4, **caractérisé par le fait que** dans le premier état distal, le deuxième élément (16) est latéralement en regard du premier élément (15), sans être en regard d'une partie du premier élément (15) attenante à son extrémité proximale (20). une partie du deuxième élément (16) saillant de l'extrémité distale (21) du premier élément (15), et **par le fait que** dans le second état proximal, le deuxième élément (16) est latéralement tout entier en regard du premier élément (15) vers son extrémité distale (21), sans être en regard d'une partie plus courte axialement du premier élément (15) attenante à son extrémité proximale (20).

6. Dispositif (1) de raccordement selon l'une quelconque des revendications 3 à 5, **caractérisé par le fait que** la chambre (18) est limitée à ses deux parties extrêmes axiales par la face transversale distale de la paroi (36) formant la tête (14) et par la face transversale d'extrémité proximale de la première bague (39) et **par le fait qu'**elle est limitée à sa périphérie par la face interne de la paroi tubulaire (19) du premier élément (15).

7. Dispositif (1) de raccordement selon la revendication 6, **caractérisé par le fait que** l'étanchéité de la chambre (18), dans le premier état distal, est assurée par l'étanchéité entre la tête (14) et son siège appartenant au premier élément (15), par l'étanchéité entre la première bague (39) et la face interne de la paroi tubulaire (19), et par l'étanchéité entre l'accessoire (3) et le troisième élément (17).

8. Dispositif (1) de raccordement selon l'une quelconque des revendications 1 à 7, **caractérisé par le fait que** le premier élément (15) comporte une paroi (19) tubulaire dont l'extrémité proximale (20) est pourvue d'un rebord (23) de fixation au récipient (2), le deuxième élément (16) comportant une paroi (24) tubulaire, insérée dans le premier élément (15), la face intérieure de la paroi tubulaire (19) et la face extérieure de la paroi tubulaire (24) comportant une combinaison d'encoche(s) (30) et d'ergot(s) (31), escamotables, disposés radialement et aptes à coopérer, formant des moyens (30, 31), amovibles de blocage de fin de course de translation axiale du deuxième élément (16) sur et par rapport au premier élément (15), sur la course C.

9. Dispositif (1) de raccordement selon la revendication 8, **caractérisé par le fait qu'**un ergot (31) est agencé de façon à être sollicité élastiquement radialement vers l'extérieur vers le fond d'une encoche (30) en regard afin de s'y loger et de façon à pouvoir être escamoté d'une encoche (30) avec laquelle il coopérait, et ainsi s'effacer élastiquement radialement vers l'intérieur pour pouvoir ensuite glisser sur la face en regard de l'élément portant la ou les encoche(s) (30).

10. Dispositif (1) de raccordement selon l'une quelconque des revendications 8 et 9, **caractérisé par le fait que** la face intérieure de la paroi tubulaire (19) du premier élément (15) et la face extérieure de la paroi tubulaire (24) du deuxième élément (16) comportent une combinaison de rainure axiale (28) et de saillie radiale (29) vers l'extérieur formant détrompeur.

11. Dispositif (1) de raccordement selon l'une quelconque des revendications 2 à 10, **caractérisé par le fait que** la bague (39, 40) comporte des canaux (43, 44), aptes à permettre le montage à l'intérieur ainsi que la fixation rigide, avec étanchéité, d'une partie fonctionnelle (11).

12. Premier ensemble (1 + 3) comprenant un dispositif (1) de raccordement selon l'une quelconque des revendications 1 à 11 et un accessoire (3) à usage unique, comportant une partie proximale active (9), fixée rigidement au troisième élément (17), la partie proximale active (9) étant logée à l'intérieur ou placée en périphérie de la chambre (18).

13. Premier ensemble (1 + 3) selon la revendication 12, **caractérisé par le fait que** la partie distale (10) de l'accessoire (3) saille de façon distale de l'extrémité distale (21) du deuxième élément (16).

14. Premier ensemble (1 + 3) selon l'une quelconque des revendications 12 et 13, **caractérisé par le fait qu'**à l'accessoire (3) est associé fonctionnellement au moins une autre partie fonctionnelle (11), placée à côté ou à proximité de l'accessoire (3).

15. Premier ensemble (1 + 3) selon la revendication 14, **caractérisé par le fait que** la partie fonctionnelle (11) est un tube d'amenée et/ou de vidange qui comporte une partie extrême proximale (12) ouverte et une partie extrême distale (13) ouverte, avec une connexion.

16. Premier ensemble (1 + 3) selon la revendication 15, **caractérisé par le fait que** la partie fonctionnelle (11) comporte au moins deux tubes, l'un d'amenée, le cas échéant de calibration, l'autre de vidange, le cas échéant de rinçage.

17. Premier ensemble (1 + 3) selon l'une quelconque des revendications 12 à 16, **caractérisé par le fait que** l'accessoire (3) est une sonde (3a).

18. Premier ensemble (1 + 3) selon l'une quelconque des revendications 12 à 16, **caractérisé par le fait que** l'accessoire (3) est un tube (3b).

19. Second ensemble (1 + 2 + 3) comprenant un ensemble (1 +3) selon l'une quelconque des revendications 12 à 18 et un récipient (2) comportant une ouverture (7) dans laquelle est monté le dispositif (1) de raccordement.

20. Second ensemble (1 + 2 + 3) selon la revendication 19, **caractérisé par le fait que** le récipient (2) est rigide et réutilisable.

21. Second ensemble (1 + 2 + 3) selon la revendication 19, **caractérisé par le fait que** le récipient (2) est souple et à usage unique, tel qu'une poche dite 2D ou 3D.

22. Second ensemble (1 + 2 + 3) selon la revendication 21, **caractérisé par le fait qu'**il est prévu des moyens rigides de maintien extérieur du récipient (2) pourvus d'un passage pour le dispositif (1) de raccordement.

23. Procédé de montage du second ensemble (1 + 2 + 3) selon l'une quelconque des revendications 19 à 22, dans lequel :
■ on dispose d'un récipient (2) vide de contenu, de l'accessoire (3), et des éléments (15, 16 et 17) constitutifs du dispositif (1) de raccordement,
■ dans une étape préliminaire, on fixe rigidement le premier élément (15) sur le récipient à l'endroit de l'ouverture (17),
■ dans une autre étape préliminaire, on insère l'accessoire (3) dans le troisième élément (17) et on le fixe rigidement à celui-ci,
■ dans une deuxième étape ultérieure, on monte l'ensemble troisième élément (17) + accessoire (3) sur et dans le deuxième élément (16),
■ dans une troisième étape, on monte l'ensemble deuxième élément (16) + troisième élément (17) + accessoire (3) sur et dans le premier élément (15), jusqu'à coopération des encoche(s) (30) et ergot(s) (31) correspondant au premier état distal.

24. Procédé de montage selon la revendication 23, **caractérisé par** une étape successive supplémentaire dans laquelle on aspire l'air (ou le gaz) se trouvant dans la chambre (18) et on la vide de celui-ci.

25. Procédé de montage selon l'une quelconque des revendications 23 et 24, **caractérisé par** une étape successive supplémentaire dans laquelle on injecte dans la chambre (18) un liquide de stockage.

26. Procédé de montage selon l'une quelconque des revendications 23 à 25, **caractérisé par** une étape successive supplémentaire dans laquelle on procède à une stérilisation du second ensemble récipient (2) + dispositif (1) de raccordement + accessoire (3).

27. Procédé de montage selon l'une quelconque des revendications 23 à 26, **caractérisé par** une étape successive supplémentaire dans laquelle on procède au conditionnement du second ensemble récipient (2) + dispositif (1) de raccordement + accessoire (3).

28. Procédé de mise en oeuvre du second ensemble (1 + 2 + 3) selon l'une quelconque des revendications 19 à 22, dans lequel on réalise des étapes opératoires liées spécifiquement au dispositif (1) de raccordement et à l'accessoire (3) comprenant les étapes opératoires suivantes :
- La chambre (18) étant vide, faire passer l'ensemble dispositif (1) de raccordement + accessoire (3) du premier état distal au second état proximal,
- Puis, mettre en oeuvre l'accessoire (3).

29. Procédé de mise en oeuvre selon la revendication 28, **caractérisé par le fait qu'**il comporte une opération supplémentaire de réglage, de contrôle ou de calibration de l'accessoire (3).

30. Procédé de mise en oeuvre selon l'une quelconque des revendication 28 et 29, **caractérisé par le fait qu'**en ce qui concerne l'étape consistant à faire passer le premier ensemble dispositif (1) de raccordement + accessoire (3) du premier état distal au second état proximal, on fait coulisser axialement l'ensemble deuxième élément (16) + troisième élément (17) + accessoire (3) sur la course C, dans le sens proximal, jusqu'à coopération des encoche(s) (30) et ergot(s) (31) correspondant au second état proximal.

31. Procédé de mise en oeuvre selon l'une quelconque des revendications 29 et 30, **caractérisé par le fait qu'**en ce qui concerne l'étape l'opération supplémentaire, il s'agit d'une calibration de l'accessoire (3) étant alors une sonde (3a) auquel est associé un tube (11), cette calibration étant réalisée au moyen de la chambre (18).

32. Procédé de mise en oeuvre selon la revendication 31, **caractérisé par le fait qu'**on réalise une calibration unique ou plusieurs calibrations, telles qu'une calibration initiale, avant que le récipient (2) ne soit empli de son contenu, une calibration finale, après que le récipient (2) a été vidangé de son contenu, et le cas échéant, une calibration intermédiaire après que le récipient (2) a été empli de son contenu et avant qu'il a été vidangé de son contenu.

33. Procédé de mise en oeuvre selon l'une quelconque des revendications 31 et 32, **caractérisé par le fait qu'**on réalise une calibration alors que l'ensemble dispositif (1) de raccordement + sonde (3a) + tube (11) est dans le premier état distal et que la chambre (18) est fermée de façon étanche.

34. Procédé de mise en oeuvre selon l'une quelconque des revendications 31 à 33, **caractérisé par le fait qu'**on réalise une calibration par les étapes suivantes :
■ on injecte d'abord une solution de calibration dans la chambre (18) fermée de façon étanche,
■ puis, la solution de calibration remplissant la chambre (18) toujours fermée de façon étanche, et la partie proximale active (9a) étant en contact avec la solution de calibration, on calibre la sonde (3a) si cela est nécessaire et comme cela est requis,
■ puis, la chambre (18) toujours fermée de façon étanche, on vidange de la solution de calibration,
■ puis on rince la chambre (18) et on la vidange.

35. Procédé de mise en oeuvre selon l'une quelconque des revendications 28 à 34, **caractérisé par** une étape opératoire préliminaire consistant à vider la chambre (18) du liquide de stockage qui y a été mis lors de la fabrication, puis, le cas échéant la rincer.

## Claims

1. A device (1) for connection to a container (2) of an accessory (3), the active proximal part (9) of which is able to communicate with the inside (4) of the container (2) through an opening (7) of the container (2), with the device including a first element (15), a second element (16), a third element (17) and a chamber (18), wherein:
■ the functions of the first element (15) consist in:
○ providing the rigid securing of the device (1) for connection to the container (2),
○ defining a passage (22) between the inside (4) and the outside (8) of the container (2),
○ providing a support for the second element (16) and an axial translation guide on an axial travel C, the two travel stops of which correspond to a first distal condition and a second proximal condition,
○ helping guiding the third element (17),
○ helping defining the chamber (18),
■ the functions of the second element (16) consist in:
○ providing a support for the third element (17), which is rigidly fixed thereto,
○ forming a mobile element axially sliding on the travel C, the two travel stops of which correspond to the first distal condition and the second proximal condition,
■ the functions of the third element (17), which a head (14) belongs to, consist in:
○ providing a support for the accessory (3), which is rigidly fixed thereto,
○ forming a mobile element axially sliding on the travel C, the two travel stops of which correspond to the first distal condition and the second proximal condition,
○ in the first distal condition, providing the sealing of the opening (7), using the head (14),
○ in the second proximal condition, providing a protection of the active proximal part (9), using the head (14),
○ helping defining the chamber (18),
■ the chamber (18) is, in the first distal condition, sealed and in the second proximal condition, in communication with the inside (4) and having the function of accommodating there inside, or of positioning in the periphery thereof, the active proximal part (9), and, if need be,
○ of placing therein a storage liquid,
○ of placing therein an accessory (3) adjustment, control or calibration liquid, or building a sealed chamber (18) wherein the accessory (3) is placed in stand-by.

2. A connection device (1) according to claim 1, **characterized in that** the third element (17) includes, in addition to the head (14), a base (34) and a body (35),
■ with the functions of the head (14) consisting in:
○ in the first distal condition, sealing the first element (15),
○ in the second proximal condition, helping protecting the active proximal part (9),
■ with the functions of the base (34) consisting in:
○ providing a support for the accessory (3), which is rigidly secured thereon,
○ sealingly providing the guiding of the sliding of the third element (17) on and with respect to the first element (15),
○ helping defining the chamber (18),
■ the body (35) including a linking part (46) provided with a thread (45) wherein the accessory (3) is inserted and at least one bearing member (47) rigidly linking the head (14) and the base (34).

3. A connection device (1) according to claim 2, **characterized in that** the base (34) includes:
■ in the proximal end part thereof, at least a first ring (39) able to sealingly and axially slide, on the outer face thereof, on and along the inner face of the tubular wall (19),
■ at the distal end part thereof, a second ring (40) able to be rigidly secured by the outer face thereof, on and against the inner face of the tubular wall (24) of the second element (16),
■ lower faces (41, 42) of the ring (39, 40), able to allow the rigid securing, or even sealing of the accessory (3).

4. A connection device (1) according to claim 3, **characterized in that** the relative position of the third element (17) with respect to the second element (16) is fixed, with the second ring (40) and the connecting part (46) being positioned in the second element (16), and with the first ring (39), the bearing members (47) and the head (14) being located outside the second element (16).

5. A connection device (1) according to any one of claims 1 to 4, **characterized in that**, in the first distal condition, the second element (16) is laterally opposite the first element (15), without being opposite a part of the first element (15) adjoining the proximal end (20) thereof, with a part of the second element (16) protruding from the distal end (21) of the first element (15) and **in that**, in the second proximal condition, the second element (16) is wholly laterally opposite the first element (15) toward the distal end (21) thereof, without being opposite an axially shorter part of the first element (15) adjoining the proximal end (20) thereof.

6. A connection device (1) according to any one of claims 3 to 5, **characterized in that** the chamber (18) is limited, at the two axial end parts thereof, by the distal transversal face of the wall (36) forming the head (14) and by the proximal end transversal face of the first ring (39) and **in that** it is limited, on the periphery thereof, by the inner face of the tubular wall (19) of the first element (15).

7. A connection device (1) according to claim 6, **characterized in that** the sealing of the chamber (18), in the first distal condition, is provided by the sealing between the head (14) and the seat thereof belonging to the first element (15), by the sealing between the first ring (39) and the inner face of the tubular wall (19), and by the sealing between the accessory (3) and the third element (17).

8. A connection device (1) according to any one of claims 1 to 7, **characterized in that** the first element (15) includes a tubular wall (19) the proximal end (20) of which is provided with an edge (23) for securing to the container (2), with the second element (16) including a tubular wall (24) inserted into the first element (15), with the inner face of the tubular wall (19) and the outer face of the tubular wall (24) including a combination of retractable notch(es) (30) and pin(s) (31) positioned radially and able to cooperate, forming removable means (30, 31) for locking the travel stops of the axial translation of the second element (16) on and with respect to the first element (15), on the travel C.

9. A connection device (1) according to claim 8, **characterized in that** a pin (31) is so arranged as to be resiliently radially actuated outwards and to the bottom of an opposite notch (30) in order to be accommodated therein, and so as to be able to be faired from a notch (30) which it cooperated with, and thus to be resiliently radially retracted inwards in order to be subsequently able to slide on the opposite face of the element bearing the notch(es) (30).

10. A connection device (1) according to any one of claims 8 and 9, **characterized in that** the inner face of the tubular wall (19) of the first element (15) and the outer face of the tubular wall (24) of the second element (16) include a combination of an axial groove (28) and a radial protrusion (29) towards the outside, forming a location notch.

11. A connection device (1) according to any one of claims 2 to 10, **characterized in that** the ring (39, 40) includes channels (43, 44) able to allow the mounting inside, as well as the rigid securing, and sealing of a functional part (11).

12. A first set (1 + 3) including a connection device (1) according to any one of claims 1 to 11, and a disposable accessory (3), including an active proximal part (9) rigidly fixed to a third element (17), with the active proximal part (9) being accommodated inside, or positioned on the periphery of the chamber (18).

13. A first set (1 + 3) according to claim 12, **characterized in that** the distal part (10) of the accessory (3) distally protrudes from the distal end (21) of the second element (16).

14. A first set (1 + 3) according to any one of claims 12 and 13, **characterized in that** another functional part (11) positioned beside or close to the accessory (3) is functionally associated with the accessory (3).

15. A first set (1 + 3) according to claim 14, **characterized in that** the functional part (11) is a feed and/or draining pipe which includes an open proximal end part (12) and an open distal end part (13), with a connection.

16. A first set (1 + 3) according to claim 15, **characterized in that** the functional part (11) includes at least two tubes, one for feeding, if need be for calibration, and the other one for draining, if need be for rinsing.

17. A first set (1 + 3) according to any one of claims 12 to 16, **characterized in that** the accessory (3) is a probe (3a).

18. A first set (1 + 3) according to any one of claims 12 to 16, **characterized in that** the accessory (3) is a tube (3b).

19. A second set (1 + 2 + 3) including a set (1 + 3) according to any of claims 12 to 18, and a container (2) including an opening (7) wherein the connection device (1) is mounted.

20. A second set (1 + 2 + 3) according to claim 19, **characterized in that** the container (2) is rigid and reusable.

21. A second set (1 + 2 + 3) according to claim 19, **characterized in that** the container (2) is flexible and disposable, such as a so-called 2D or 3D bag.

22. A second set (1 + 2 + 3) according to claim 21, **characterized in that** rigid means for the external holding of the container (2) provided with a passage for the connection device (1).

23. A method for mounting the second set (1 + 2 + 3) according to any one of claims 19 to 22, wherein:
■ a container (2) containing nothing, the accessory (3) and the elements (15, 16 and 17) composing the connection device (1) are provided,
■ in a preliminary step, the first element (15) is rigidly fixed to the container, where the opening (17) is provided,
■ in another preliminary step, the accessory (3) is inserted into the third element (17) and rigidly fixed thereto,
■ in a second subsequent step, the third element (17) + accessory (3) set is mounted on and in the second element (16),
■ during a third step, the second element (16) + third element (17) + accessory (3) set is mounted on and in the first element (15), up to the cooperation of the notch(es) (30) and the pin(s) (31) corresponding to the first distal condition.

24. A mounting method according to claim 23, **characterized by** an additional successive step, wherein the air (or gas) present in the chamber (18) is sucked and the chamber is emptied thereof.

25. A mounting method according to any one of claims 23 and 24, **characterized by** an additional successive step, wherein a storage liquid is injected into the chamber (18).

26. A mounting method according to any one of claims 23 to 25, **characterized by** an additional successive step, wherein the second container (2) + connection device (1) + accessory (3) set is sterilized.

27. A mounting method according to any one of claims 23 to 26, **characterized by** an additional successive step, wherein the second container (2) + connection device (1) + accessory (3) set is packaged.

28. A method for implementing the second set (1 + 2 + 3) according to any one of claims 19 to 22, wherein operational steps specifically linked to the connection device (1) and to the accessory (3) are carried out and include the following operational steps:
- with the chamber (18) being empty, the connection device (1) + accessory (3) set changes from the first distal condition to the second proximal condition,
- then the accessory (3) is implemented.

29. A method of implementation according to claim 28, **characterized in that** it includes an additional operation of adjusting, checking or calibrating the accessory (3).

30. A method of implementation, according to any one of claims 28 and 29, **characterized in that** during the step consisting in switching the first connection device (1) + accessory (3) set from the first distal condition to the second proximal condition, the second element (16) + third element (17) + accessory (3) set axially slides along the stroke C, in the proximal direction, up to the cooperation of the notch(es) (30) and the pin(s) (31) corresponding to the second proximal condition.

31. A method of implementation, according to any one of claims 29 and 30, **characterized in that**, as far as the step of additional operation is concerned, the calibration relates to an accessory (3), which is then a probe (3a), to which a tube (11) is associated, with such calibration being carried out using the chamber (18).

32. A method of implementation, according to claim 31, **characterized in that** only one or several calibration(s) is/are carried out, such as an initial calibration, before the container (2) is filled with the content thereof, a final calibration, after the container (2) has been emptied of its contents, and if need be, an intermediate calibration after the container (2) has been filled with its content and before it has been emptied of its content.

33. A method of implementation, according to any one of claims 31 and 32, **characterized in that** a calibration is carried out whereas the connection device (1) + probe (3a) + tube (11) set is in the first distal condition and the chamber (18) is sealed.

34. A method of implementation, according to any one of claims 31 to 33, **characterized in that** a calibration is carried out through the following steps:
■ a calibration solution is first injected into the sealed chamber (18),
■ then, with calibration solution filling the still sealed chamber (18) and the active proximal part (9a) being in contact with the calibration solution, the probe (3a) is calibrated if necessary and as required,
■ then, with the still sealed chamber (18), the calibration solution is emptied,
■ then the chamber (18) is rinsed and emptied.

35. A method of implementation, according to any one of claims 28 to 34, **characterized by** a preliminary operational step consisting in emptying the chamber (18) of the storage liquid which was placed there during the production, and to rinse it, if need be.

## Patentansprüche

1. Vorrichtung (1) für den Anschluss an einen Behälter (2) eines Zubehörteils (3), dessen aktives proximales Teil (9) mit dem Inneren (4) des Behälters (2) in Verbindung gesetzt werden kann über eine Öffnung (7) des Behälters (2), wobei die Vorrichtung ein erstes Element (15), ein zweites Element (16), ein drittes Element (17) und eine Kammer (18) umfasst, bei dem:
- das erste Element (15) die folgenden Funktionen hat:
. die steife Befestigung der Anschlussvorrichtung (1) am Behälter zu gewährleisten,
. einen Durchlass (22) zu definieren zwischen dem Inneren (4) und dem Äußeren (8) des Behälters (2),
. als Träger zu dienen für das zweite Element (16) und als axiale Translationsführung auf einem axialen Weg C, dessen beiden Endpunkte einem ersten distalen Zustand und einem zweiten proximalen Zustand entsprechen,
. dazu beizutragen als Führung für das dritte Element (17) zu dienen,
. dazu beizutragen, die Kammer (18) abzugrenzen,
- des zweite Element (16) die folgenden Funktionen hat:
. als Träger zu dienen für das dritte Element (17), das steif an ihm befestigt ist,
. ein mobiles Element zu bilden, das axial auf einem Weg C mobil ist, dessen beiden Endpunkte dem ersten distalen Zustand und dem zweiten proximalen Zustand entsprechen,
- das dritte Element (17), zu dem ein Kopf (14) gehört, die folgenden Funktionen hat:
. als Träger zu dienen für das Zubehörteil (3), das steif an ihm befestigt ist,
. ein mobiles Element zu bilden, das axial auf einem Weg C mobil ist, dessen beiden Endpunkte dem ersten distalen Zustand und dem zweiten proximalen Zustand entsprechen,
. im ersten distalen Zustand dank des Kopfs (14) das dichte Schließen der Öffnung (7) zu gewährleisten,
. im zweiten proximalen Zustand dank des Kopfs (14) einen Schutz des aktiven proximalen Teils (9) zu gewährleisten,
. dazu beizutragen, die Kammer (18) abzugrenzen,
- die Kammer (18) im ersten distalen Zustand fest geschossen ist und im zweiten proximalen Zustand in Verbindung mit dem Inneren (4) steht, und die Funktion hat, das aktive proximale Teil (9) im Inneren aufzunehmen oder an der Peripherie anzuordnen, und gegebenenfalls,
. eine Lagerflüssigkeit aufzunehmen,
. eine Einstell-, Kontroll- oder Kalibrierungsflüssigkeit des Zubehörteils (3) aufzunehmen, oder eine dicht geschlossene Kammer (18) zu bilden, in der das Zubehörteil (3) bis zur Verwendung aufbewahrt wird.

2. Anschlussvorrichtung (1) gemäß Anspruch 1, **dadurch gekennzeichnet, dass** das dritte Element (17) außer dem Kopf (14) eine Basis (34) und einen Körper (35) aufweist, wobei
- der Kopf (14) die folgenden Funktionen hat:
. im ersten distalen Zustand das erste Element (15) fest zu verschließen,
. im zweiten proximalen Zustand dazu beizutragen, das aktive proximale Teil (9) zu schützen,
- die Basis (34) die folgenden Funktionen hat:
. als Träger zu dienen für das Zubehörteil (3), das steif mit ihm verbunden ist,
. die Führung des Gleitens mit Dichtheit zu gewährleisten, des dritten Elements (17) auf dem und in Bezug auf das erste Element (15),
. dazu beizutragen, die Kammer (18) abzugrenzen,
- der Körper (35) ein Verbindungsteil (46) mit einer Bohrung (45) umfasst, in der das Zubehörteil (3) aufgenommen wird, und mindestens ein Tragorgan (47), das den Kopf (14) steif mit der Basis (34) verbindet.

3. Anschlussvorrichtung (1) gemäß Anspruch 2, **dadurch gekennzeichnet, dass** die Basis (34) folgendes umfasst:
- an ihrem proximalen Endteil, mindestens einen ersten Ring (39), der axial mit Dichtheit mit seiner Außenseite an und entlang der Innenseite der rohrförmigen Wand (19) verschiebbar ist,
- an ihrem distalen Endteil, einen zweiten Ring (40), der mit seiner Außenseite an der und gegen die Innenseite der rohrförmigen Wand (19) des zweiten Elements (16) steif befestigt werden kann,
- Innenseiten (41, 42) des Rings (39, 40), die die steife Befestigung mit Dichtheit des Zubehörteils (3) ermöglichen können.

4. Anschlussvorrichtung (1) gemäß Anspruch 3, **dadurch gekennzeichnet, dass** die relative Position des dritten Elements (17) in Bezug auf das zweite Element (16) fest ist, wobei sich der zweite Ring (40) und das Verbindungsteil (46) im zweiten Element (16) befinden, wobei der erste Ring (39), die Tragorgane (47) und der Kopf (14) außerhalb des zweiten Elements (16) sitzen.

5. Anschlussvorrichtung (1) gemäß einem der Ansprüche 1 bis 4, **dadurch gekennzeichnet, dass** das zweite Element (16) im ersten distalen Zustand seitlich dem ersten Element (15) gegenübersitzt, ohne sich gegenüber einem Teil des ersten Elements (15) zu befinden, das sich an seinem proximalen Ende (20) anschließt, wobei ein Teil des zweiten Elements (16) über das distale Ende (21) des ersten Elements (15) hinausragt, und dadurch, dass sich das zweite Element (16) im zweiten proximalen Zustand komplett seitlich gegenüber dem ersten Element (15) befindet, in Richtung dessen distalen Endes, ohne gegenüber einem axial kürzeren Teil des ersten Elements (15) zu sitzen, das sich an dessen proximalen Ende (20) anschließt.

6. Anschlussvorrichtung (1) gemäß einem der Ansprüche 3 bis 5, **dadurch gekennzeichnet, dass** die Kammer (18) an ihren beiden axialen Endteilen begrenzt wird von der distalen Querseite der den Kopf (14) bildenden Wand (36) und von der proximalen Querseite am Ende des ersten Rings (39), und dadurch, dass sie an ihrer Peripherie begrenzt wird durch die Innenseite der rohrförmigen Wand (19) des ersten Elements (15).

7. Anschlussvorrichtung (1) gemäß Anspruch 6, **dadurch gekennzeichnet, dass** die Dichtheit der Kammer (18) im ersten distalen Zustand gewährleistet wird durch die Dichtheit zwischen dem Kopf (14) und seinem zum ersten Element (15) gehörenden Sitz, durch die Dichtheit zwischen dem ersten Ring (39) und der Innenseite der ringförmigen Wand (19) und durch die Dichtheit zwischen dem Zubehörteil (3) und dem dritten Element (17).

8. Anschlussvorrichtung (1) gemäß einem der Ansprüche 1 bis 7, **dadurch gekennzeichnet, dass** das erste Element (15) eine rohrförmige Wand (19) aufweist, deren proximales Ende (20) mit einer Befestigungskante (23) am Behälter versehen ist, wobei das zweite Element (16) eine rohrförmige Wand (24) aufweist, die in das erste Element (15) eingefügt ist, wobei die Innenseite der rohrförmigen Wand (19) und die Außenseite der rohrförmigen Wand (24) eine Kombination von Kerbe(n) (30) und Nocke(n) (31) umfasst, die einziehbar und radial angeordnet sind und abnehmbare Mittel (30, 31) bilden, die zusammenwirken können, um das Ende des axialen Translationswegs des zweiten Elements (16) auf dem und in Bezug auf das erste Element (15) auf dem Weg C zu blockieren.

9. Anschlussvorrichtung (1) gemäß Anspruch 8, **dadurch gekennzeichnet, dass** ein Nocken (31) so angeordnet ist, dass er radial elastisch nach Außen in Richtung auf den Boden einer gegenüberliegenden Kerbe (30) belastet werden kann, um dort aufgenommen und von einer Kerbe (30) eingezogen zu werden, mit der er zusammenwirkte, um sich so radial elastisch nach Innen zurückzuziehen, um danach an der Seite gegenüber dem Element gleiten zu können, das die Kerbe(n) (30) trägt.

10. Anschlussvorrichtung (1) gemäß einem der Ansprüche 8 und 9, **dadurch gekennzeichnet, dass** die Innenseite der rohrförmigen Wand (19) des ersten Elements (15) und die Außenseite der rohrförmigen Wand (24) des zweiten Elements (16) eine Kombination umfassen aus einer axialen Rille (28) und einem radialen Vorspruch (29) nach Außen, der eine Unverwechselbarkeitsvorrichtung bildet.

11. Anschlussvorrichtung (1) gemäß einem der Ansprüche 2 bis 10, **dadurch gekennzeichnet, dass** der Ring (39, 40) zwei Kanäle (43, 44) aufweist, die die Innenmontage sowie die steife Befestigung mit Dichtheit eines Funktionsteils (11) ermöglichen können.

12. Erste Baugruppe (1 + 3) bestehend aus der Anschlussvorrichtung (1) gemäß einem der Ansprüche 1 bis 11, und einem Einweg-Zubehörteil (3) mit einem aktiven proximalen Teil (9), das steif am dritten Element (17) befestigt ist, wobei das aktive proximale Teil (9) innerhalb oder an der Peripherie der Kammer (18) angeordnet ist.

13. Erste Baugruppe (1 + 3) gemäß Anspruch 12, **dadurch gekennzeichnet, dass** das distale Teil (10) des Zubehörteils (3) das distale Ende (21) des zweiten Elements (16) distal überragt.

14. Erste Baugruppe (1 + 3) gemäß Anspruch 12 und 13, **dadurch gekennzeichnet, dass** das Zubehörteil (3) funktional verbunden ist mit mindestens einem anderen Funktionsteil (11), das neben dem oder in der Nähe des Zubehörteils (3) angeordnet ist.

15. Erste Baugruppe (1 + 3) gemäß Anspruch 14, **dadurch gekennzeichnet, dass** das Funktionsteil (11) ein Zu-und/oder Ableitungsrohr ist, das ein proximales offenes Endteil (12) und ein distales offenes Endteil (13) mit einer Verbindung umfasst.

16. Erste Baugruppe (1 + 3) gemäß Anspruch 15, **dadurch gekennzeichnet, dass** das Funktionsteil (11) mindestens zwei Rohre, d.h. ein Zuleitungsrohr, gegebenenfalls für die Kalibrierung, und ein Ableitungsrohr, gegebenenfalls für die Spülung, umfasst.

17. Erste Baugruppe (1 + 3) gemäß einem der Ansprüche 12 bis 16, **dadurch gekennzeichnet, dass** das Zubehörteil (3) eine Sonde (3a) ist.

18. Erste Baugruppe (1 + 3) gemäß einem der Ansprüche 12 bis 16, **dadurch gekennzeichnet, dass** das Zubehörteil (3) ein Rohr (3b) ist.

19. Zweite Baugruppe (1 + 2 + 3), bestehend aus einer Baugruppe (1 + 3) gemäß einem der Ansprüche 12 bis 18, und einem Behälter (2) mit einer Öffnung (7), in die die Anschlussvorrichtung (1) eingebaut ist.

20. Zweite Baugruppe (1 + 2 + 3) gemäß Anspruch 19, **dadurch gekennzeichnet, dass** der Behälter (2) steif und wieder verwendbar ist.

21. Zweite Baugruppe (1 + 2 + 3) gemäß Anspruch 19, **dadurch gekennzeichnet, dass** der Behälter (2) flexibel und nur einmal verwendbar ist, wie ein so genannter 2D-oder 3D-Beutel.

22. Zweite Baugruppe (1 + 2 + 3) gemäß Anspruch 21, **dadurch gekennzeichnet, dass** außerhalb des Behälters (2) steife Haltemittel vorgesehen sind, die einen Durchlass für die Anschlussvorrichtung (1) aufweisen.

23. Montageverfahren der zweiten Baugruppe (1 + 2 + 3) gemäß einem der Ansprüche 19 bis 22, bei dem man:
- einen leeren Behälter (2) ohne das Zubehörteil (3) und die Elemente (15, 16 und 17), die die Anschlussvorrichtung bilden, bereitstellt,
- in einem ersten Schritt das erste Element (15) steif am Behälter im Bereich der Öffnung (17) befestigt,
- in einem weiteren Vorschritt das Zubehörteil (3) in das dritte Element (17) einfügt und es steif an diesem befestigt,
- in einem zweiten späteren Schritt die Baugruppe drittes Element (17) + Zubehörteil (3) am und im zweiten Element (16) anbringt,
- in einem dritten Schritt die Baugruppe zweites Element (16) + drittes Element (17) + Zubehörteil (3) am und im ersten Element (15) anbringt, bis die Kerbe(n) (30) und Nocke(n) (31) zusammenwirken, um dem ersten distalen Zustand zu entsprechen.

24. Montageverfahren gemäß Anspruch 23, **gekennzeichnet durch** einen zusätzlichen Folgeschritt, bei dem man die Luft (oder das Gas) aus der Kammer (18) absaugt und sie davon entleert.

25. Montageverfahren gemäß einem der Ansprüche 23 und 24, **gekennzeichnet durch** einen zusätzlichen Folgeschritt, bei dem man eine Lagerflüssigkeit in die Kammer (18) spritzt.

26. Montageverfahren gemäß einem der Ansprüche 23 bis 25, **gekennzeichnet durch** einen zusätzlichen Folgeschritt, bei dem man die zweite Baugruppe Behälter (2) + Anschlussvorrichtung (1) + Zubehörteil (3) sterilisiert.

27. Montageverfahren gemäß einem der Ansprüche 23 bis 26, **gekennzeichnet durch** einen zusätzlichen Folgeschritt, bei dem man die zweite Baugruppe Behälter (2) + Anschlussvorrichtung (1) + Zubehörteil (3) konditioniert.

28. Einsatzverfahren der zweiten Baugruppe (1 + 2 + 3) gemäß einem der Ansprüche 19 bis 22, bei dem man die operativen Schritte durchführt, die spezifisch die Anschlussvorrichtung (1) und das Zubehörteil (3) betreffen, mit den folgenden operativen Schritten:
- bei leerer Kammer (18) realisiert man den Übergang der Baugruppe Anschlussvorrichtung (1) + Zubehörteil (3) aus dem ersten distalen Zustand in den zweiten proximalen Zustand,
- danach setzt man das Zubehörteil (3) ein.

29. Einsatzverfahren gemäß Anspruch 28, **dadurch gekennzeichnet, dass** es eine zusätzliche Einstell-, Kontroll- oder Kalibieroperation des Zubehörteils (3) umfasst.

30. Einsatzverfahren gemäß einem der Ansprüche 28 und 29, **dadurch gekennzeichnet, dass** man bezüglich des Schritts, bei dem die erste Baugruppe Anschussvorrichtung (1) + Zubehörteil (3) aus dem ersten distalen Zustand in den zweiten proximalen Zustand versetzt, die Baugruppe zweites Element (16) + drittes Element (17) + Zubehörteil (3) axial über den Weg C verschiebt, in proximaler Richtung, bis die Kerbe(n) und die Nocke(n) (31) zusammenwirken, um dem zweiten proximalen Zustand zu entsprechen.

31. Einsatzverfahren gemäß einem der Ansprüche 29 und 30, **dadurch gekennzeichnet, dass** es sich, was den Schritt zusätzliche Operation anbetrifft, um eine Kalibrierung des Zubehörteils (3) handelt, das dabei eine Sonde (3a) ist, mit der ein Rohr (11) verbunden ist, wobei diese Kalibrierung anhand der Kammer (18) erfolgt.

32. Einsatzverfahren gemäß Anspruch 31, **dadurch gekennzeichnet, dass** man eine einzige Kalibrierung oder mehrere Kalibrierung vornimmt, wie beispielsweise eine Anfangskalibrierung, bevor der Behälter (2) mit seinem Inhalt gefüllt wird, eine Endkalibrierung, nachdem der Behälter (2) von seinem Inhalt geleert wurde, und gegebenenfalls eine Zwischenkalibrierung, nachdem der Behälter (2) mit seinem Inhalt gefüllt und bevor er von seinem Inhalt geleert wurde.

33. Einsatzverfahren gemäß einem der Ansprüche 31 bis 32, **dadurch gekennzeichnet, dass** man eine Kalibrierung vornimmt, während sich die Baugruppe Anschlussvorrichtung (1) + Sonde (3a) + Rohr (11) im ersten distalen Zustand befindet und die Kammer (18) dicht verschlossen ist.

34. Einsatzverfahren gemäß einem der Ansprüche 31 bis 33, **dadurch gekennzeichnet, dass** man eine Kalibrierung in den folgenden Schritten vornimmt:
- zuerst spritzt man eine Kalibrierlösung in die dicht verschlossene Kammer (18) ein,
- wenn dann die Kalibrierlösung die stets dicht verschlossene Kammer (18) füllt, und das aktive proximale Teil (9a) mit der Kalibrierlösung in Kontakt steht, kalibriert man die Sonde (3a), wenn dies notwendig und wie es erforderlich ist,
- dann entleert man bei stets dicht verschlossener Kammer (18) die Kalibrierlösung,
- danach spült man die Kammer (18) und leert sie.

35. Einsatzverfahren gemäß einem der Ansprüche 28 bis 34, **gekennzeichnet durch** einen vorherigen operativen Schritt, der darin besteht, die Kammer (18) von der Lagerflüssigkeit zu leeren, die dort bei der Fabrikation eingefüllt wurde, und sie gegebenenfalls zu spülen.
